# EUROPEAN PATENT SPECIFICATION

(11) **EP 2 200 537 B1**
(45) Date of publication and mention of the grant of the patent: **11.11.2015**
(21) Application number: 08785830.4
(22) Date of filing: 05.09.2008
(51) Int. Cl.: A61K 38/00, A61L 27/56, A61L 27/42, A61L 27/54, A61L 27/58, C07K 14/495, A61F 2/28

(54) **CALCIUM PHOSPHATE BASED DELIVERY OF GROWTH AND DIFFERENTIATION FACTORS TO COMPROMISED BONE**
CALCIUMPHOSPHAT-BASIERTE ABGABE VON WACHSTUMS- UND DIFFERENZIERUNGSFAKTOREN AN BEEINTRÄCHTIGTEN KNOCHEN
ADMINISTRATION AVEC DU PHOSPHATE DE CALCIUM DE FACTEURS DE CROISSANCE ET DE DIFFÉRENCIATION À UN OS FRAGILISÉ

(30) Priority: 05.09.2007 US 970258 P; 30.10.2007 US 928889; 21.11.2007 EP 07022543
(43) Date of publication of application: 30.06.2010
(73) Proprietor: Friedrich-Schiller-Universität Jena, 07745 Jena (DE); Universitätsklinikum Jena, 07743 Jena (DE)
(72) Inventor: VENBROCKS, Rudolf, A., 97762 Hammelburg (DE); KINNE, Raimund, W., 07743 Jena (DE); JANDT, Klaus, D., 07751 Jena (DE); BOSSERT, Joerg, 07616 Buergel (DE); HORTSCHANSKY, Peter, 07749 Jena (DE); SCHMUCK, Klaus, 07552 Gera (DE)
(74) Representative: Jany, Peter
(86) International application number: PCT/EP2008/007248
(87) International publication number: WO 2009/030483

(56) References cited:
- EP-A- 1 604 693
- DE-A1- 19 647 853
- US-A1- 2005 170 012

## Description

### Field of the Invention

This invention relates to compositions comprising growth and differentiation factor(s) (GDF(s)) and calcium phosphate (CaP) material as a carrier ("CaP-GDF"). The invention, in particular, relates to biocompatible, osteoinductive and/or osteogenetic compositions for use in, e.g., bone regeneration, osseous augmentation, fracture prevention, tissue repair, and reinforcement in bone fractures, bone implants, and prostheses.

### Background of the Invention

The publications and other materials, including patents and accession numbers, are used herein to illustrate the invention and, in particular, to provide additional details respecting the practice.

The repair, regeneration, and augmentation of bone tissue in fractures have been subject to research for many years. With the increase of osteoporosis and other bone weaknesses in aging populations, the demands on bone regeneration technology, in particular implant technology, have heightened. In an osteoporotic patient, the bone mass may be reduced considerably. When the reduction is at about 40%, the facture threshold has been reached. About 50% of such patients obtain fractures due to regular everyday activities, with fractures in the femoral neck and the spine being particularly common. In those patients, the natural bone regenerative processes are compromised, thus resulting in poor healing of fractures. Also implants that might work well in young adults, might not work well in such patients. Due to the relative stiffness of many available implants, in patients with compromised regenerative processes, the surrounding bone might, after implantation, be exposed to stresses from the implant that cause follow-up fractures (Baroud and Bohner, Joint Bone Spine 73, 144-150 (2006). Currently 67% of all osteoporotic patients with the most common type of vertebral body implants suffer from such a follow-up fracture within one year.

Several members of the transforming growth factor ß (TGF-ß)/bone morphogenetic protein (BMP) superfamily, in particular, BMP-2 and BMP-7, have been employed in orthopedic and trauma surgery to accelerate and enhance bone regeneration in individuals with fractures, so as to improve fracture healing or to initiate spinal fusion. However, the use of these factors also bears promise in the treatment of individuals with compromised bone metabolism, such as osteoporotic or elderly patients. Soluble BMP released from rapidly degrading carriers, such as collagen, have been favored. For example, human recombinant BMP-2 produced in mammalian cell lines with collagen from cattle as its carrier is marketed under the name InFuse® (Medtronic Sofamor Darnek, USA) and InductOs® (Wyeth, USA). Commercially available BMP-7, which is marketed under the name Osigraft® (Stryker Biotech, USA), is also bound to collagen from cattle.

Next to growth-enhancing proteins such as BMPs, other avenues have been pursued in reconstructive surgery to replace damaged tissue. Thus, cartilage regeneration systems in which isolated chondrocytes are injected into a damaged area in the context of a polymer scaffold have been used (see, e.g., Atala et al., J. Urol. 150:747 (1993); Freed et al., J. Cell. Biochem. 51:257 (1993) and references cited therein). Similar seeded scaffold systems have been studied in the context of bone repair. Here, osteoblasts are utilized in conjunction with polymeric or ceramic supports (see, e.g., Elgendy et al., Biomat. 14:263 (1993); Ishaug et al., J. Biomed. Mater. Res. 28:1445 (1994)).

Polymethylmethacrylate (PMMA) is a bone cement widely used in orthopedics to remodel lost or damaged bone. As a synthetic polymer, it is supplied in a powder with liquid methylmethacrylate (MMA). When mixed, a dough-like cement results that gradually hardens. While PMMA is biologically compatible, it has been considered a possible carcinogen and has been linked to cardiopulmonary events due to hypertension. It has a Young's modulus (elasticity modulus) between callous bone and cortical bone, namely about 2000 MPa, and is not resorbed upon bone-remodeling.

Calcium phosphate ceramics have high biocompatibility and exhibit good to excellent affinity for protein reagents (see, e.g., Ijntema et al., Int. J. Pharm. 112:215 (1994); Itokazu et al., J. Orth. Res. 10:440 (1992); Shinto et al., J. Bone Joint Surg. 74-B:600 (1992); and Uchida et al., J. Orth. Res. 10:440 (1992)). Most of these calcium phosphate materials consist of prefabricated, sintered hydroxyapatite in either granule or block forms. However, these preparations often have properties that impair their effective use in the repair of skeletal defects. Granulate may not stick together and may otherwise be structurally weak. Blocks, such as hydroxyapatite blocks, may be hard to model, which render them difficult to use in implantation, which requires the formation of shapes determined by the individual defect.

In general, all of these products are ceramics, produced by high temperature sintering and are often biologically non-resorbable. Other porous, non-resorbable materials are based on coral material and are marketed under the name HA RESORB and HAPSET in the dental market.

US. Pat. Nos. Re. 33,221 and Re. 33,161 to Brown and Chow teach preparation of calcium phosphate remineralization compositions and a finely crystalline, non-ceramic, gradually resorbable hydroxyapatite (HA) material based on calcium phosphate compositions, which are precursors of hydroxyapatite (HA) and are biologically compatible. These compositions have, in contrast to many other calcium phosphate biomaterials, two advantageous properties: (1) self-hardening to form a mass with sufficient strength for many medical and dental applications, and (2) when implanted in bone, the material resorbs slowly and is completely replaced by new bone formation with no loss in the volume or integrity of the tissue that receives the implant.

Hydroxyapatite cements were shown to have desirable biomechanical characteristics in vertebral body structures reinforced with such cements (Seino et al., J Orthop. Sci. 8:50-54 (2003).

A similar calcium phosphate system based on tetracalcium phosphate (TTCP) and monocalcium phosphate (MCP) or its monohydrate form (MCPM) and which forms hydroxyapatite (HA) is disclosed by Constantz et al. (U.S. Pat. Nos. 5,053,212 and 5,129,905).

In DE 196 47 853, Paulista et al. describe a material comprising as a matrix preferably phase pure, highly porous (20 to 60% of the volume) alpha and beta tricalcium phosphate (TCP) with a highly interconnecting microporous microstructure, which can house proteins, such as members of the TGF-ß superfamily that are released upon TCP resorption. The authors explain that undefined compositions of TCP with HA or other CaP phases, i.e. not phase pure TCP, may lead to negative biomedical properties such as activation of macrophages or infiltration of soft tissue.

EP 1 604 693 to Schütz et al. describes an in-situ hardening paste comprising a homogeneous polymer matrix of a biodegradable water-insoluble polymer, and a water-soluble polymeric plasticizer with an organic or inorganic, water-insoluble, solid filing material (e.g., calcium phosphate). The paste serves as a delivery system for an active agent (e.g., an osteoinductive or cartilage inductive protein of the TGF-ß family or BMP subfamily). The paste is produced via a viscous liquid of a water-soluble and a water-insoluble polymer to which the active agent is either directly mixed or is added as a coating of the filler (e.g., calcium phosphate). The polymer material constitutes a considerable portion of the composite carrier material, generally more than 20%. The polymeric phase of Schütz et al. contains pores with a pore size in the nm range.

US Patent Publication 2005/0170012 to Dalal et al. discloses a porous ß-tricalcium phosphate material for bone implantation. The multiple pores in the porous TCP body are preferably separate discrete voids of 20 µm to 500 µm and are not interconnected, since such porous ceramics are considered brittle and are not capable of being easily shaped during an operation. Dalal et al. discloses that excessively large pore size and high porosity of the ceramic material can lead to excessive resorption rates, thus, preventing the matrix from providing a scaffold for the newly synthesized bone, whereas small pore size and low porosity of the ceramic material will lead to low resorption rates causing encapsulation of matrix particles in the new bone. However, the authors do not teach to combine a ceramic material having an excessive resorption rate with another ceramic material having a low(er) resorption rate. A spherical pore structure is said to provide the porous body with the necessary mechanical strength during the period that new bone is being synthesized, thus preventing the bone from fracturing during this period.

U.S. Pat. Nos. 5,650,176; 5,676,976; 5,683,461; 6,027,742 and 6,117,456 to Lee et al., describe a type of calcium phosphate composition that comprises an amorphous calcium phosphate as a reactant, a promoter and an aqueous liquid to form a hardening paste. This system provides a bioactive ceramic material that is biocompatible, bioresorbable and workable for long periods of time at room temperature. The bioactive ceramic material may be formed at low temperatures, is readily formable and/or injectable and yet can harden to high strength upon further reaction. The bioactive ceramic material contains so called poorly crystalline apatitic calcium phosphate solids with calcium-to-phosphate (Ca/P) ratios comparable to naturally occurring bone minerals and has stiffness and fracture roughness similar to natural bone.

US Pat. Appl. No. 20020187104 to Li et al., describes calcium phosphate delivery vehicles for osteoinductive proteins, such as BMP-2 and BMP-7. An everesent agent is designed to prevent the formation of a unitary monolithic structure. Li's biocompatible and bioresorbable calcium phosphate material self-granulates and disperses into hardened macroparticles subsequent to administration. The particles are designed to be sufficiently large to prevent an inflammation reaction. The osteoinductive proteins which may be dispersed homogenously throughout the volume of the individual particle are designed to stimulate osteogenic activity *in vivo.*

The bone morphogenic factors, BMP-2 and BMP-7, are currently being used in clinical practice in combination with collagen from cattle. However, BMP-2 and BMP-7 are associated with side-effects such as the induction of tumors of haemapoietic cells, ectopic ossification, and systemic antibody formation (Harwood et al., Expert Opin Drug Saf 4(1): 75-89 (2005)). GDF-5 defines a distinct structural and functional subgroup of BMPs and shares 40-50% protein-sequence identity (38% sequence identity on the mRNA level) with BMP-2 and BMP-7 (osteogenic protein-1, or OP-1). GDF-5 is expressed in the prechondrogenic mesenchyme and can induce/promote the formation of the initial condensations in micromass cultures, while e.g., the widely used BMP-2, BMP-3 and activin act at the later steps of chondrogenesis (P. H. Francis-West, Development 126, 1305-1315 (1999)). GDF-3's sequence identity on the mRNA level to BMP-2 is, with 20%, even lower. While other members of the BMP family are expressed at different embryonic stages and in different regions of developing bone, GDF-3 is expressed in the osteogenic zone of newly calcifying bone (Jones Mol. Endo 6(11), 1961-68 (1992)).

Experiments with GDF-5 provided, in comparison to BMP-2, varying results, but showed that certain collagen matrixes appeared to influence the activity of GDF-5 (Spiro, Biochem Soc. 28(4) 362-368 (2000); Spiro, Anat. Record 263, 388-395 (2001)). Purified human GDF-5 showed bone-forming potential when reconstituted with allogenic inactivated bone matrix (Hoetten, Growth Factors 13, 65-74 (1996)). When used with rat bone residue, recombinant GDF-5 was considerably less active than BMP-7 (OP-1). It was also reported that GDF-5 when used with hydroxyapatite (HA) showed by itself very little osteogenic activity, but that it displayed, when administered in conjunction with culture-expanded bone marrow mesenchymal stem cells (MSC), a considerable osteogenic response (J. Biomed Mater Res A 68(1), 168-76 (2004)).

Thus, there is a need for a composition that comprises GDF or a functional fragment thereof, and a vehicle conducive for unfolding GDF's osteogenic potential. The composition should, in a preferred embodiment, contain carrier material that is biocompatible and readily resorbable. GDFs should preferably be released at a rate conductive for osteogenesis. Such a composition is preferably injectable, promotes homogeneous distribution of GDF throughout the resorbable material, and thus permits controlled release of the active substance. And finally, the composition should form a material displaying an advantageous resorption rate that is adjusted to osteogenesis, thus facilitating cell migration and infiltration for secretion of extracellular bone matrix as well as vascularisation. Advantageously, a high surface area may be provided for enhanced resorption and release of active substance, as well as increased cell-matrix interaction.

### Brief Description of the Figures

Fig. 1 shows in (A) a nucleotide sequence encoding the MPB-GDF-5 fusion protein shown in (B), which will, subsequent to cleavage, result in the mature GDF-5 protein shown in (C).
Fig. 2 shows the relative increase of bone density in lumbar vertebral bodies upon administration of hydroxyapatite (HA), either alone or in combination with rhBMP-2 and rhGDF-5 ("rhBMP").
Fig. 3 depicts the results of compression strength measurements upon administration of hydroxyapatite (HA), either alone or in combination with rhBMP-2 at an optimal dosage after 3 months.
Fig. 4 depicts the results of compression strength measurements upon administration of hydroxyapatite (HA), either alone or in combination with rhGDF-5 at a minimal dosage after 9 months.
Fig. 5 shows the relative increase of bone volume in lumbar vertebral bodies upon administration of hydroxyapatite (HA), either alone or in combination with rhBMP-2, both at the injection channel (a) and at a remote area (here at a distance of about 10 mm) (b) after 9 months.
Fig. 6 shows the relative increase of bone volume in lumbar vertebral bodies upon administration of hydroxyapatite (HA), either alone or in combination with rhGDF-5, both at the injection channel (a) and at a remote area (here at a distance of about 10 mm) (b) after 9 months.
Fig. 7 shows the increase of the diameter of bone trabeculae [µm] in lumbar vertebral bodies upon administration of hydroxyapatite (HA), either alone or in combination with rhBMP-2, both at the injection channel (a) and at a remote area (here at a distance of about 10 mm) (b) after 9 months.
Fig. 8 shows the increase of the diameter of bone trabeculae [µm] in lumbar vertebral bodies upon administration of hydroxyapatite (HA), either alone or in combination with rhGDF-5, both at the injection channel (a) and at a remote area (here at a distance of about 10 mm) (b) after 9 months.
Fig. 9 shows the relative increase in osteoblast surface [%] in lumbar vertebral bodies upon administration of hydroxyapatite (HA), either alone or in combination with rhBMP-2, both at the injection channel (a) and at a remote area (here at a distance of about 10 mm) (b) after 9 months.
Fig. 10 shows the relative increase in osteoblast surface [%] in lumbar vertebral bodies upon administration of hydroxyapatite (HA), either alone or in combination with rhGDF-5, both at the injection channel (a) and at a remote area (here at a distance of about 10 mm) (b) after 9 months.

### Summary of the Invention

The present invention is directed at a composition as represented by claim 1 for delivering recombinant growth and differentiation factor(s) comprising a bioresorbable calcium phosphate material; and GDF-5 and/or GDF-3, as a biologically active agent. The composition is preferably cell-free, is injectable. The recombinant GDF-5 is, in one embodiment of the invention, a non-glycosylated human recombinant GDF-5. The composition may comprise as biologically active agent at least one further osteogenic and/or osteoinductive protein selected from bone morphogenic proteins and/or further GDFs and/or a transforming growth factors. The recombinant GDF-5 may comprise SEQ ID NO: 1: MNSMDPEST. The composition is provided in a liquid form for purposes of injection. Preferably the calcium phosphate material is the primary component of the composition, i.e. is the quantitatively largest (major) part/portion of the composition.

The calcium phosphate material may be a calcium phosphate such as, but not limited to, monocalciumphosphate anhydrite, calciumhydrogenphosphate dihydrate, dicalciumphosphate dihydrate, brushite, calciumhydrogenphosphate anhydrite, hydroxyapatite (HA), α-tricalcium phosphate, ß-tricalcium phosphate, fluorapatite, or a combination thereof.

The calcium phosphate material is composed of materials that are resorbed at different speeds, that is, have differing resorption rates. As a result, the calcium phosphate material, upon hardening, comprises phases that resorb at different rates.

In general the resorption rate (resorption kinetic) of the material may be (should be) adapted to the needs of a specific individual patient (e.g. age, metabolic conditions, kind of disease of that patient). As discussed below, less quickly resorbing particles, such as HA particles can be embedded in faster resorbing phases such as α-tricalcium phosphate (α-TCP) and/or ß-tricalcium phosphate (ß-TCP) and/or dicalcium phosphate dihydrate (DCPD), and/or (mineral) brushite (CaHPO₄ • 2H20). In general all calcium-phosphates known in the art (see below table 1, published as table 2 by Dorozhkin SV (2007) J Mater Sci 42:1061-1095) may be used to create the desired phases in the hardened product. Table 1 below provides measurements of the solubility of different calcium phosphates (calcium phosphate species). As can be seen from this table, HA displays low solubility values, both at 25 °C and 37 °C, while the values for α-TCP and ß-TCP are much higher than those of HA, while the values for DCPD are in turn much higher than those of α-TCP and ß-TCP.

**Table 1**

| **Compound** | **Formula** | **Solubility at 37 °C, -log (Kₛ)** |
|---|---|---|
| Dicalcium phosphate dihydrate (DCPD), mineral brushite | CaHPO₄ • 2H₂O | 6.63 |
| Dicalcium phosphate anhydrous (DCPA), mineral monetite | CaHPO₄ | 7.02 |
| Octacalcium phosphate (OCP | Ca₈(HPO₄)₂(PO₄)₄ • 5H₂O | 95.90 |
| α-Tricalcium phosphate (α-TCP) | α-Ca₃(PO₄)₂ | 25.50 |
| ß-Tricalcium phosphate (ß-TCP) | ß-Ca₃(PO₄)₂ | 29.50 |
| Calcium-deficient hydroxyapatite (CDHA) | Ca₁₀-x(HPO₄)ₓ(PO₄)₆₋ₓ | ~ 85.10 |
| Hydroxyapatite (HA or OHAp) | Ca₁₀(PO₄)₆(OH)₂ | 117.20 |
| Fluorapatite (FA or Fap) | Ca₁₀(PO₄)₆F₂ | 119.20 |
| Tetracalcium phosphate (TTCP or TetCP), mineral hilgenstockite | Ca₄(PO₄)₂O | 37-42 |

In general the resorption rate(s)/ kinetic(s) of the calcium phosphate material may be adapted to the specific requirements of a specific individual patient. That is the formulation of a composition intended for application to a specific patient may be individualized as optimally as possible, in particular the calcium phosphate material components (portions) may be elected with regard to their specific resorption rate(s)/kinetic(s) and with regard to the age, the metabolic conditions and the kind of disease of the individual patient.

Accordingly, the invention is also directed at a method for preparing a porous, bioresorbable, GDF-releasing calcium phosphate cement according to claim 1 for use in repair, regeneration and/or augmenting of bone tissue, especially individually adapted to a selected individual patient. Said method comprises (a) providing a bioresorbable calcium phosphate material, which contains several different calcium phosphates (calcium phosphate species), in combination with , GDF-5 and/or GDF-3 as a biologically active agent; and (b) mixing said components so that pores are formed upon hardening to produce a porous, bioresorbable, GDF-releasing calcium phosphate cement. Especially for an individualized therapeutical application said calcium phosphate material and optionally further additives such as gas forming agent(s) and/or bioerodible polymeric structure(s) should be provided in amounts that are adapted to (calibrated against) each other, and that, after mixing and hardening, result in a GDF-releasing calcium phosphate cement with resorption rate(s) and GDF-releasing rates that are specifically adjusted to the age, the metabolic conditions and the kind of disease of a selected individual patient.

As the person skilled in the art will readily understand, adjusting the composition of a particular portion, will allow adjustment of the material to the needs of an individual patient.

The calcium phosphate may be a powder or granule which hardens when mixed with a reactive agent such as phosphoric acid. The hardened material comprises an inorganic matrix formed by the reactive agent and the calcium phosphate, e.g., powder or granule. The matrix may contain calcium phosphate that did not participate in the reaction with the reactive agent.

The calcium phosphate material may, upon hardening, be strongly bioresorbable. Also, the calcium phosphate material may, upon hardening, comprise interconnecting pores. In general the resulting calcium-phosphate-cement contains pores which affect its strength (stability) and resorption kinetics. The desired porosity and thus the strength and resorption kinetics depend on the designated medical field of the application. For example, a calcium-phosphate-cement according to the invention that is intended for use in spinal column surgery may have a porosity of about 40% and a compression strength of about 35 MPa and may contain (as final composition) 55% dicalcium phosphate dihydrate (DCPD) and 45% ß-tricalcium phosphate (ß-TCP), wherein in the DCPD portion the average pore size is less than 5 µm and in the ß-TCP portion the average pore size is between 2 and 50 µm, and wherein pores with a size of about 1 µm numerically predominate over pores with a size of about 200 µm.

The composition which comprises calcium phosphate as main part/portion further comprises a bioerodible polymeric structure such as a polysaccharide, peptide, protein, synthetic polymer, natural polymer, a synthetic or natural copolymer, or combinations thereof which may provide one or more further phase(s) referred to herein as "polymeric or polymer phase(s)". In such an embodiment, the hardened bioresorbable calcium phosphate material (calcium phosphate cement) preferably contains less than about 15%, preferably less than about 10%, more preferably less than about 5% of the polymer phase(s). These polymer phase(s) are either part of the inorganic matrix or form a perculation microstructure with the phosphate material. The polymer phase may consist of various materials.

The polymeric structure is/are microscaffold(s) having a porous structure. Such microscaffolds increase the strength (stability) of the CaP-cement but do not influence the porosity of the CaP-cement. The microscaffold(s) may be between 200 µm and 2 mm, preferably 500 µpm and 1 mm in size and/or may have a pore channel diameters of about 30 to 500 µm, preferably of about 100 to 200 µm. The microscaffold(s) preferably have a size that is less than the size of the inner diameter of the syringe used for injection. However, the microscaffold(s) may have diameters that exceed the inner diameter of the syringe used for injection. The polymeric structure/microscaffold may favorably be made from chitosan and/or a poly-lactic-acid/ poly-e-caprolactone (PLA-PCL) copolymer but may also be made of any other degradable polymer. It may further be coated with a biocompatible nanocrystalline calcium phosphate such as, but not limited to, monocalciumphosphate anhydrite, calciumhydrogenphosphate dihydrate, dicalciumphosphate dihydrate, brushite calciumhydrogenphosphate anhydrite, hydroxyapatite, α-tricalcium phosphate, ß-tricalcium phosphate, fluorapatite, or a combination thereof.

A preferred embodiment of the invention comprises the use of microscaffolds which can be infiltrated by one or more of the calcium phosphates materials so that they are ultimately filled with CaP-cement (referred to herein as "CaP filled microscaffolds" in contrast to empty microscaffolds, so-called foam beads). In addition these microscaffolds may be (preferably homogenously) coated with biomimetic hydroxyapatite (so-called nanoapatite) which provides a better connection between the microscaffold polymers and the CaP-cement.

The calcium phosphate material is upon hardening, strongly bioresorbable and comprises porous phases that resorb at different rates e.g., a first phase that resorbs at first rate and which may comprise pores having an average diameter of less than 1 µm and which may be in particles the first phase essentially consist of or comprises, and a second phase that resorbs at a second rate which exceeds that of said first rate. Any pores that may be part of the first phase may preferably include the second phase.

The compostion may, in a certain embodiment, comprise a gas-forming agent such as, but not limited to, hydrogen, carbon dioxide, air, nitrogen, helium, oxygen, or argon.

The invention also discloses a composition which, upon hardening, is a cement having a compression strength of more than 4 MPa such as between about 5 MPa and about 200 MPa, preferably of more than about 10 MPa, more than about 20 MPa, more than about 30 MPa, preferably between about 20 MPa and about 200 MPa, between about 20 MPa and 100 MPa or between 20 and 40 MPa. Said composition comprises at least a growth and differentiation factor, such as GDF-5 and GDF3, and a biocompatible and bioresorbable CaP material.

Important for practical application (i.e. surgery) is a fast initial compression strength of the cement. The following hardening periods may be suitable: binding period 9-11 minutes; about 6 MPa (Megapascal) compression strength after 20 minutes; complete (final) compression strength after 1-5 days. Such hardening periods allow a first mobilisation of the patient after 24 hours.

A calcium phosphate cement according to the invention with a desired compression strength of about 35 MPa is already described above as example which is intended for use in spinal column surgery.

The invention also discloses a method for furthering bone formation comprising administering to a bone location, such as a fractured and/or osteoporotic bone of a mammal, the above composition in a bone-forming effective amount. The composition may be administered in one or more doses of less than 100 µg. Said composition, upon administration, may result in an increase of at least about 20%, 25%, 30%, 35%, or 40% in bone volume at said bone location. This method may also include measuring the bone mineral density (BMD) of the respective bone and determining a lower threshold value of said BMD.

The invention is also directed at a kit comprising:
(a) the bioresorbable calcium phosphate material as described herein in one container;
(b) the above biologically active agent in the same or a separate container; and
(c) in a further container, instructions of how to use (a) and (b).

The biologically active agent may be admixed in a buffer solution or may be admixed with the bioresorbable calcium phosphate material. The kit may further comprise tools for kyphoplasty, vertebroplasty or a porous pouch. The pouch can also comprise, consist of, or essentially consist of a resorbable material, for example, Chitosan, which can, for example, be manufactured by using electro-spinning. The pore sizes of the resorbable pouch may be 1-1000 µm, preferably 100-500 µm and even more preferably 200-250 µm. The pouch may be made of a polyester material, preferably PET. The pores of the pouch may have a diameter of less than about 1 mm.

The invention is also directed at a method for preparing a porous, bioresorbable, GDF-releasing calcium phosphate cement according to claim 1 comprising:
- providing a combination of a bioresorbable calcium phosphate material and a GDF as a biologically active agent and
- mixing said combination so that pores are formed upon hardening to produce a porous, bioresorbable, GDF-releasing calcium phosphate cement.

Said pores may be elongated and have a diameter of about 100 µm to about 1 mm, preferably 100 µm to about 200 µm. The pores may have a length of more than 1 mm. Alternatively or additionally, the pores may be spherical pores with a diameter of less than 1 µm. The method may further comprise providing a cavity at the bone location, inserting a porous pouch into the cavity and administering the composition into said porous pouch, e.g., via a single passage to said cavity.

The invention discloses a composition which, upon hardening, is a cement that comprises several phases having different resorption rates, wherein said composition comprises a growth and differentiation factor, such as GDF-5 and GDF3, and a biocompatible and bioresorbable CaP material and further microscaffolds with or without biomimetic apatite (nanoapatite).

The invention also discloses a composition which, upon hardening, is a cement that comprises pores, wherein said composition comprises a growth and differentiation factor, such as GDF-5 and GDF3, and a biocompatible and bioresorbable CaP material and further comprising a gas-forming agent, and/or further comprising a bioerodible polymeric structure, preferably in form of a microscaffold having porous structure, and/or further comprising a porous pouch into which the CaP material is filled. The CaP material may be a powder.

### Detailed Description of Various and Preferred Embodiments of the Invention

The present invention is directed at compositions as in claim adapted for use in the repair, regeneration, and augmentation of bone tissue. The composition comprises a growth and differentiation factor, which is GDF-5 and or GDF-3, and a biocompatible and bioresorbable calcium phosphate material. Preferably, the composition is cell-free. Upon hardening, the composition provides a resorbable material for new bone growth. A gas-forming agent may be included in the composition to promote formation of a desired pore structure or control porosity. The growth and differentiation factor(s) stimulate osteoinductive/osteogenic activity of present or infiltrating progenitor or other cells. The compositions are in particular useful for osseous augmentation and regeneration of bone tissue, for example, in osteopenic or osteoporotic bone, as well as for tissue repair and reinforcement in bone fractures, dental implants, bone implants and prostheses, and the like.

Growth and differentiation factor-5 (GDF-5; also known as MP52, CDMP-1 or BMP-14) and growth and differentiation factor-3 (GDF-3; also known as Vgr-2) are members of the heterogeneous transforming growth factor ß (TGF-ß)/bone morphogenetic protein (BMP) superfamily. Members of this family are known for their role in the developmental events that dictate skeletal patterning. GDF-5 defines a distinct structural and functional subgroup of BMPs and shares 40-50% protein-sequence homology (38% sequence identity on the RNA level) with BMP-2 and BMP-7 (osteogenic protein-1, or OP-1). GDF-5 has a high binding affinity for the BMP receptor ALK-6 and is expressed in the prechondrogenic mesenchyme and can induce/promote the formation of the initial condensations in micromass cultures, while e.g., the widely used BMP-2, BMP-3 and activins act at the later steps of chondrogenesis (P. H. Francis-West, Development 126, 1305-1315 (1999)).

Recombinant human (rh) GDF-5 could also be shown to stimulate mesenchyme aggregation and chondrogenesis. However, in contrast to BMP-2, osteoblastic MC3T3-E1 cells, which serve as osteogenic markers, did not respond to rh GDF-5, suggesting that GDF-5 may be relatively specific for chondrogenesis. Also, the receptor-binding profiles of GDF-5 are more limited than those of BMP-2 and BMP-7 and these receptors display a more restrictive tissue distribution (Spiro, Anat. Record 263,388-395 (2001)). GDF-5 and BMP-2 also show completely different temporal and spatial expression patterns in developing limbs, with GDF-5 having been attributed a role in the early stage of chondrogenesis and chondrocyte development. In cell culture studies, recombinant human GDF demonstrated to be considerably less potent in osteogenesis relative to other BMPs, in particular BMP-2 (Hoetten, Growth Factors 13, 65-74 (1996), (Depprich et al., Mund Kiefer Gesichtschir., 9(6): 363-8 (2005) as well as BMP-7 (OP-1) (Erlacher, J Bone Mineral Res. 13(3), 383-392 (1998)).

GDF-3's sequence identity on the RNA level to BMP-2 is, with 20% even lower than that of GDF-5. While other members of the BMP family are expressed at different embryonic stages and in different regions of developing bone, GDF-3 is expressed in the osteogenic zone of newly calcifying bone (Jones Mol. Endo 6(11), 1961-68 (1992)).

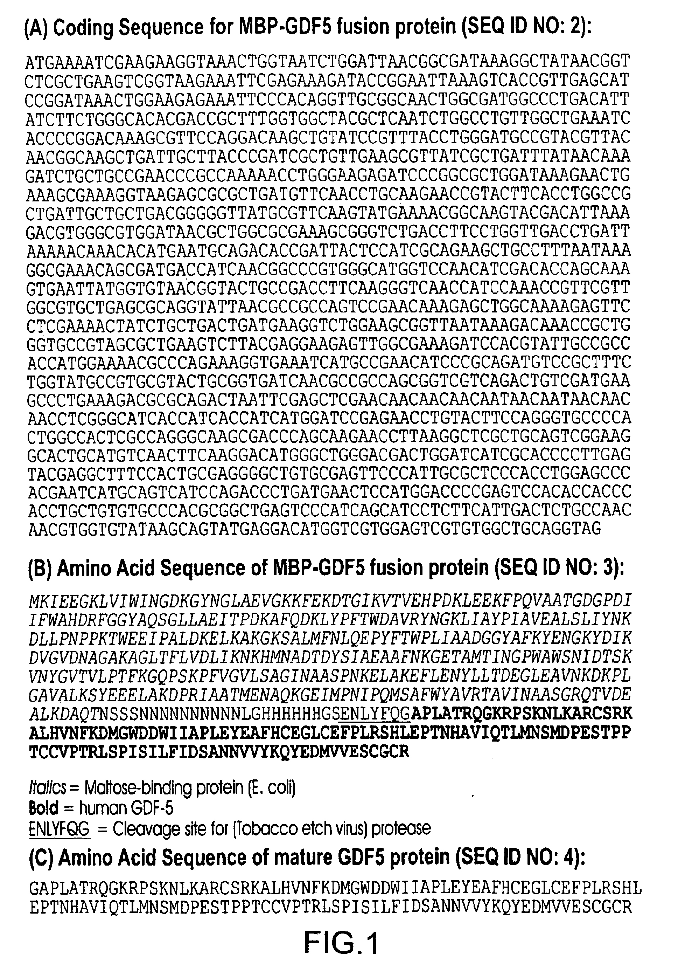

WO 93/1609 (see also U.S. Patent 6,120,760) describes a coding sequence of GDF-5. Generally, GDF-5 is expressed as a precursor which is composed of an amino terminal signal sequence and a carboxyterminal sequence of about 100 amino acids, which after cleavage presents the mature protein. The *Gdf*5 gene from human can be found under accession no. X80915 (PRI 04-MAY-2005), the amino acid sequence can be found under Swiss-Prot: P43026 (modified 2007-08-21). Fig. 1 (A) shows a coding region for GDF-5 and Fig. 1 (B) the amino acid sequence of a precursor fusion protein of GDF-5, while Fig. 1 (C) shows the amino acid sequence of a mature GDF-5.

Any "biologically active agent" according to the present invention furthers (ergo is an osteogenic biologically active agent) or induces (ergo is an osteoinductive biologically active agent) bone growth when provided as part of the composition of the present invention. Of particular interest are osteogenic and/or osteoinductive materials such as bone morphogenetic proteins such as BMP-2, BMP-4, BMP-5, BMP-6, BMP-7, BMP-10, in particular growth and differentiation factors (GDFs) such as GDF-6 and GDF-7, but in particular, e.g., recombinant human GDF-5 or GDF-3, transforming growth factors (e.g., TGF-ß), and various other organic species known to further and/or induce bone formation. The biologically active agent may also comprise, in certain embodiments, bone-forming cells or bone-forming precursor cells.

A "growth and differentiation factor" (GDF) according to the present disclosure is any GDF, in particular GDF-5 and/or GDF-3, or a functional fragment or functional variant thereof that furthers and/or induces osteogenesis (are osteogenic and/or osteoinductive) when provided in the composition and method of the present disclosure. A "functional fragment" can, for example, comprise terminal and/or internal omissions compared to a wild type or a recombinant GDF produced from a cDNA of a wild type, as long as it furthers or induces osteogenesis when provided as part of the composition, kit and/or method of the present invention. A functional variant can, for example, contain one or more point mutations compared to the wild type. A functional variant may also be a functional fragment, that is, comprise one or more point mutations as well as terminal and/or internal omissions as long as the functional variant furthers and/or induces osteogenesis when provided as part of the composition and/or method of the present invention.

Functional fragments and functional variants having this function, but whose functionality is reduced to about 70%, about 80%, or about 90% of a wild type, or enhanced by about 10%, about 20%, or about 30% compared to a wild type or recombinant GDF produced from a cDNA of a wild type, are also within the scope of the present invention. A functional fragment preferably comprises SEQ ID No: 1: MNSMDPEST. The GDF-5 and/or GDF-3 or functional fragment may be at least 50%, 60%, 70%, 75%, 80%, 85%, 90%, 95%, 96%, 97%, 98%, or 99% identical to the respective wild type sequence, that is, have these sequence identities (in %) with this wild type sequence.

The term "sequence identity" refers to a measure of the identity of nucleotide sequences or amino acid sequences. In general, the sequences are aligned so that the highest order match is obtained. "Identity" per se has recognized meaning in the art and can be calculated using published techniques. (See, e.g.: Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). While there exist a number of methods to measure identity between two polynucleotide or polypeptide sequences, the term "identity" is well-known to skilled artisans (Carillo, H. & Lipton, D., SIAM J Applied Math 48:1073 (1988)).

Whether any particular nucleic acid molecule is at least 50%, 60%, 70%, 75%, 80%, 85%, 90%, 95%, 96%, 97%, 98%, or 99% identical to a nucleic acid sequence encoding a specified GDF or a functional fragment thereof, can be determined conventionally using known computer programs such as DNAsis software (Hitachi Software, San Bruno, Calif.) for initial sequence alignment followed by ESEE version 3.0 DNA/protein sequence software (cabot@trog.mbb.sfu.ca) for multiple sequence alignments. An alternative sequence analysis program is provided under the name VECTOR NTI by Invitrogen.

Whether the amino acid sequence is at least 50%, 60%, 70%, 75%, 80%, 85%, 90%, 95%, 96%, 97%, 98%, or 99% identical to, for instance a wild-type ICD amino acid sequences, or a subportion thereof, can be determined conventionally using known computer programs such the BESTFIT program (Wisconsin Sequence Analysis Package, Version 8 for Unix, Genetics Computer Group, University Research Park, 575 Science Drive, Madison, Wis. 53711). BESTFIT uses the local homology algorithm of Smith and Waterman, Advances in Applied Mathematics 2:482-489 (1981), to find the best segment of homology between two sequences.

When using DNAsis, ESEE, BESTFIT or any other sequence alignment program to determine whether a particular sequence is, for instance, 95% identical to a reference sequence according to the present invention, the parameters are set such that the percentage of identity is calculated over the full-length of the reference nucleic acid sequence and that gaps in homology of up to 5% of the total number of nucleotides in the reference sequence are allowed.

The GDF according to the present invention, such as GDF-5 may be recombinantly produced, or, alternatively, purified from a protein composition. Preferably, the GDF of the present invention is produced recombinantely, e.g., in mammalian cell cultures. Bacterially-produced GDF is, however, in a certain embodiment of the invention, even more preferred. The GDF may be non-glycosylated and may have relatively low solublity. In certain embodiments the GDF remains on the carrier for, e.g., one year up to a lifetime, preferably one to ten years. As the person skilled in the art will appreciate, in certain applications, a shorter retention time may be preferable, e.g., about 1 year or less, while in other applications, a longer retention time may be preferable. Longer retention times allow in certain embodiments for low dosage administration regimes, such as from about 100 µg or less, up to 1 mg.

The administration may be a single administration or may be repeated two or three times. Low dosage regimes reduce the negative side effects that have been associated with the use of BMPs such as BMP-2 and BMP-7, e.g., the induction of tumors of haemapoietic cells, ectopic ossification, and systemic antibody formation (Harwood et al., Expert Opin Drug Saf 4(1): 75-89 (2005)). The GDF may also be only partially glycosylated, that is less glycosylated than the respective wild type GDF. The GDF may be homodimeric, or may form a heterodimer with another BMP or with other members of the TGF-ß superfamily, such as activins, inhibins and TGF-ß-1 (e.g., a heterodimer composed of one monomer each of a BMP and a related member of the TGF ß superfamily). Examples of such heterodimeric proteins are described for example in Published PCT Patent Application WO 93/09229 (see also, e.g., U.S. Patent 6,593,109).

The compositions of the present invention may further comprise additional agents such as the Hedgehog, Frazzled, Chordin, Noggin, Cerberus, and Follistatin proteins. These families of proteins are generally described in Sasai et al., Cell 79:779-790 (1994) (Chordin); PCT Patent Publication WO 94/05800 (Noggin); and Fukui et al., Devel. Biol. 159:131-139 (1993) (Follistatin). Hedgehog proteins are described in WO 96/16668; WO 96/17924; and WO 95/18856. The biologically active agent may also include other soluble receptors, such as the truncated soluble receptors disclosed in PCT patent publication WO 95/07982. From the teaching of WO 95/07982, one skilled in the art will recognize that truncated soluble receptors can be prepared for numerous other receptor proteins, which are also encompassed within the present invention.

The amount of GDF useful herein is an amount effective to stimulate increased osteogenic and/or osteoinductive activity of infiltrating progenitor or other cells (a "bone formation effective amount"). In clinical applications such as for the treatment or prevention of fractures of a patient in need thereof, the effective amount will depend upon the size and nature of the defect being treated or the anticipated chance of fracture. Generally, the amount of protein to be delivered is in the range of from 1 µg to 100 mg, preferably less than 10 mg, less than 1 mg, less than 750 µg, or less than 500 µg. Most preferably, the CaP-GDF, is administered at relatively low doses of GDF, e.g., 500 µg or less of GDF, such as 400 µg, 300 µg, 200 µg, 100 µg, in particular less than 100 µg, including less than 75 µg, 50 µg, or 25 µg.

As described in the Examples, the GDF comprising calciumphosphate (CaP-GDF) composition may be injected into a fractured vertebral body, e.g., of a person having osteoporosis. The treatment regime may comprise in a preferred embodiment a single administration step or may comprise a series of administrations. Preventatively, the CaP-GDF composition can be injected into vertebral bodies adjacent to the fractured vertebral body to prevent post-implant fractures at such adjacent vertebral bodies.

Such an injection can either be performed after the treatment of the fractured vertebral body has progressed to a point at which new bone has formed, e.g., the total bone volume has increased about 10%, about 20%, about 30%, about 40%, or about 50% at a local area or alternatively, e.g., two weeks or more, e.g., four week to six weeks, two or three months, or longer after the initial treatment regime of the fractured vertebral body has been completed. However, as the person skilled in the art will recognize, the timing of such a subsequent injection will depend on factors such as the overall health of the patient, the condition of the bone material of the patient, with heavily damaged bone material advocating for a sooner second administration. Alternatively, the preventative injection of the CaP-GDF composition is performed at the time the fractured vertebral body is treated. CaP-GDF may also be administered purely preventatively, e.g., solely based on a high likelihood of fracture.

The likelihood of fracture can, e.g., be determined, e.g., by the standard deviation of the bone mineral density (BMD) measured in a weakened bone similar to that that will be determinative for bisphosphonate administration. The value at which treatment is warranted depends on a multitude of factors such as age, gender, previous fractures, nicotin consumption, etc. For example, treatment may be warranted in an otherwise healthy woman over 75 or a man over 85 at a standard deviation as low as -2.0, but in man between 60 and 70 only at a standard deviation of about -4.5. However, if the man in the latter example smokes, the value may be lowered to -3.5. In a patient with rheumatoid arthritis, which is subject to a prolonged steroid treatment, a preventative treatment might be warranted at a standard deviation of -1.5. In particular during a preventative treatment regime, the CaP-GDF may be administered in combination with other bone strengtheners, e.g., bisphosphonates such as alendronate or the parathyroid hormone fragment teriparatid.

In a preferred embodiment, kits are provided comprising the elements of the present invention. For example, such a kit may comprise, in one container, the calcium phosphate material, optionally in dried form, optionally admixed with the biologically active agent which may also be provided in dried, e.g., freeze-dried, form. Another container may include instructions how to admix and/or use the materials in the first container. The biologically active agent may alternatively be provided in a further separate container. Here the biologically active agent may be provided in dried form or may be in solution that, e.g., may also comprise a buffer material. In certain embodiments the kit may also comprise administration tools for, e.g., kyphoplasty. Those tools may, e.g., include injection needles specifically adjusted to the components of the composition that shall be administered to a patient.

The CaP-GDF composition, kits, and methods of the present invention are, in a preferred embodiment, used in conjunction with vertebroplasty and/or kyphoplasty. In a classical form of these two approaches to bone regeneration, PMMA cement is injected into the fractured vertebral body to render it mechanically more resistant. In kyphoplasty pressurized balloons are used to increase the height of the affected vertebral body. Published US Patent application 20060195115 describes for example, an apparatus for kyphoplasty. US Patent 6,423,083 describes a removable balloon for use in compressing cancellous bone and marrow against the inner cortex of bones whether the bones are fractured or not. Published US Patent application 20060182780 describes a method of manufacturing a resorbable balloon designed to contain bone cement for vertebroplasty or kyphoplasty applications. As the person skilled in the art will appreciate, the compositions, kits, and methods can also be employed in the context of spondylodesis.

The conversion reaction that produces eventually the hardened material is initiated by adding distilled water to a mixture of the dry precursor components to form a thick hydrated precursor in the form of a paste or putty. "Injectable" hydrated precursor material has a consistency appropriate for delivery through, e.g., an 18 or 9 gauge injection needle. In particular, the injectable material may have a consistency that allows delivery through an injection needle having an inner diameter of 1 to 10 mm, preferably 2 to 3 mm, such as 2.2 mm. The hydrated precursor material undergoes conversion to a hardened calcium phosphate, optionally with the assistance of a promoter material such as dicalcium phosphate dihydrate (DCPC). Preferably the hardening is completed in less than 12 hours, preferably 5 hours, more preferably about 5 minutes to about 60 minutes, and even more preferably about 10 minutes to about 30 minutes.

The time course of conversion may be adjusted to the surgical procedure employed, but should, in any event, be fast enough to have surgical utility. The hardening does, in a preferred embodiment, proceed *in situ.* However, alternatively, the material may, e.g., be pre-hardened outside the body, loaded with the desired biologically active agent, and implanted at a later time. This approach is useful in those situations where custom shapes are not essential, and where production of large numbers of implants is desired.

As used herein, a "calcium phosphate material" means a synthetic bone substitute material comprising calcium phosphate as the primary component. Suitable calcium phosphate-based materials are well known in the art and include, without limitation, amorphous calcium phosphate, hydroxyapatite, tricalcium phosphate, such as α/ß-tricalcium phosphate, and fluorapatite. In a preferred embodiment, the calcium phosphate material is an apatitic calcium phosphate solid having a calcium-to-phosphate (Ca/P) ratio comparable to naturally occurring bone minerals. However, in certain embodiments, the calcium phosphate material will be "calcium deficient", with a calcium to phosphate ratio of less than or equal to 1.5 as compared to the ideal stoichiometric value of approximately 1.67 for hydroxyapatite. Such materials may be produced using a combination of amorphous calcium phosphate as a reactant, a promoter, and an aqueous liquid to form a hardening paste. In an alternative embodiment, the calcium phosphate material is produced by solid-state acid-base reaction of crystalline calcium phosphate reactants to form crystalline calcium phosphate solids. In a further embodiment, monocalciumphosphate hydrate is mixed with tricalcium phosphate, such as ß-tricalcium phosphate and water, to produce brushite and water.

A "gas-forming agent" refers to a gaseous substance or a substance, which produces bubbling, foaming, or liberation of a gas. These substances include, but are not limited to, hydrogen, carbon dioxide, air, nitrogen, helium, oxygen, or argon.

As used herein, "amorphous" (also known as "X-ray amorphous") means a material with significant amorphous character. Significant amorphous character contemplates greater than 75% amorphous content, preferably greater than 90% amorphous content, and is characterized by a broad, featureless X-ray diffraction pattern.

The term "biocompatible", as used herein, means that the material does not elicit a substantial detrimental response in the host. There is always concern, when a foreign object is introduced into a living body, that the object will induce an immune reaction, such as an inflammatory response that will have negative effects on the host. For example, although hydroxyapatite is generally considered to be "biocompatible", significant inflammation and tissue necrosis have been observed when crystalline hydroxyapatite microcarriers are inserted intramuscularly in animals (see, for example, Jintema et al., Int. J. Pharm. 112:215 (1994)).

"Bioresorbable" refers to the ability of a material to be resorbed *in vivo.* The resorption process involves elimination of the original implant materials through the action of body fluids, enzymes, or cells. Resorbed calcium phosphate may, for example, be redeposited as bone mineral, be otherwise reutilized within the body, or excreted. "Strongly bioresorbable," as that term is used herein, means that at least 80% of the total mass of material implanted intramuscularly, subcutaneously, or into bone is resorbed within one year. In preferred embodiments, the material will be resorbed within nine months, six months, three months, and in certain embodiments, one month.

A composition of the present invention is said to be "cell-free" if, prior to administration, it does not contain any bone-forming cells, such as progenitor cells, stem cells, and/or osteoblasts.

An "effective amount" of a gas-forming agent is an amount sufficient to effect the formation of a desired porosity and a desired pore structure upon hardening, and will depend upon the calcium phosphate material being used. Generally, the amount of gas-forming agent is added in a range of from about 1 to 90 percent by weight, preferably about 1 to 50 percent by weight, and more preferably about 10 to 40 percent by weight.

A "porosity" that is desirable to further resorption generally entails pores having a diameter of more than about 100 µm, preferably about 100 to about 200 µm. However, larger pores such pores having a diameter of, e.g, 500 µm are also within the scope of the present invention. However, in certain embodiments the hardened material has, additionally or alternatively, small pores, such as pores of 1 µm or less, which, while allowing a certain degree of accessiblity, confer a high degree of mechanical strength. In terms of "pore structure" interconnecting pores that further resorption by facilitating cell migration throughout the material and provide access for unobstructed vascularisation are preferred.

As used herein, "macroporous" refers to a hardened calcium phosphate material having pores of sufficient diameter to permit cell migration and infiltration. The macroporous material formed in accordance with the present invention may have a pore diameter of greater than 10 µm or greater than 30 µm, more preferably between about 30 and 200 µm, and most preferably between about 50 and 100 µm in diameter. The macroporous material of the present invention preferably facilitates cell migration and infiltration for secretion of extracellular bone matrix, as well as enhancing cell-matrix interactions. In a preferred embodiment, pores of the hardened calcium phosphate material are interconnected to further enhance cell migration and infiltration throughout.

As used herein, "microporous" of preferred embodiments refers to a hardened calcium phosphate material having pores of a diameter of less than about 100 µm, generally between about 1 and about 100 µm. "Nanoporous" of preferred embodiments refers to a hardened calcium phosphate material having pores of a diameter of less than 1 µm.

An "effective amount" of a growth and differentiation factor or a composition comprising such a growth and differentiation factor is an amount sufficient to stimulate increased osteogenic activity of present or infiltrating progenitor or other cells ("bone formation effective amount"). The amount will depend upon the size and nature of the defect being treated, as well as the composition of the calcium phosphate material being employed. Generally, the amount of biologically active agent to be delivered is in a range from about from 1 µg to 100 mg, preferably less than 10 mg, less than 1 mg, less than 750 µg or less than 500 µg. Most preferably, the CaP-GDF, is administered at relatively low doses of GDF, e.g., 500 µg or less of GDF, such as 400 µg, 300 µg, 200 µg, 100 µg, in particular less than 100 µg, including less than 75 µg, 50 µg, or 25 µg.

An "effective amount" of an additional material such as a polymeric structure is an amount sufficient to impart the desired mechanical or chemical property to the composite.

"Hardening" refers to the process by which the malleable calcium phosphate composition is transformed into a hardened calcium phosphate material. The calcium phosphate material/the composition comprising the calcium phosphate material is considered to be "hardened" when it is a substantially non-formable solid. Such a hardened calcium phosphate material has minimal compressibility and tends to undergo plastic as opposed to elastic deformation. A hardened composition according to the present invention is also referred to herein as a "cement" or "matrix".

The "elastic modulus" is a parameter that represents the stiffness of a material. The elastic modulus is used herein to represent a force per unit surface area and is therefore given in Pascal (1 bar = 10⁵ Pa), or Megapascal (1 MPa). Cortical bone has a higher elastic modulus (e.g., about 18,000 MPa) than cancellous bone (e.g., about 150 MPa). The elastic modulus is heavily influenced by age, presence of osteoporosis, skeletal site, etc. Treatment with the composition of the present invention may result in an about 2 fold, 5 fold, 6 fold, 7 fold, 8 fold, 9 fold, 10 fold, or higher increase in the elastic modulus. A desirable elastic modulus in bone treated with the composition described herein will depend on the skeletal site treated. However, in certain embodiments, depending on the bone material treated , the desirable elastic modules of the treated bone is about 50 to about 1500 MPa, preferably about 100 to about 150 MPa or about 500 to about 1200 MPa (see also, Baroud and Boher, Joint Bone Spine 73:144-150 (2006).

"Compression strength" refers generally to the amount of pressure that can be applied to a material without causing damage. To determine the compression strength, pressure is generally axially applied. Usually the parameter "compression strength" will be determined *in vitro* via special compression strength testing machines (test control units) known in the art (e.g. manufactured and/or sold by INSTRON, Pfungstadt, DE, ZWICK, Ulm, DE or Kennesaw, GA, USA; KÖGEL, Engelsdorf, DE). The testing will be carried out according the international norm ISO 5833.

The amount of pressure at which the material is no longer able to withhold and breaks, defines the compression strength of the material. Cortical bone has been reported to have a compression strength of about 100 MPa, while osteoporotic cancellous bone has been reported to have a compression strength of about 4 MPa Baroud and Boher, Joint Bone Spine 73:144-150 (2006).

The transitional phrase "consisting essentially of" limits the scope of a claim to the specified materials or steps "and those that do not materially affect the basic and novel characteristic(s)" of the claimed invention. In general the term "essentially" as used herein has the meaning of "completely or almost completely".

### The Calcium Phosphate Material

Calcium phosphate component of the present invention may be any biocompatible, calcium phosphate material known in the art (see, e.g, U.S. Patent 6,027,742 and U.S. Pat. No. 5,650,176). Hardening of the calcium phosphate material may be achieved by any one of a variety of methods and using any suitable starting components. For example, the hardening of calcium phosphate material may be initiated using a combination of a calcium phosphate material as a reactant, a promoter and an aqueous liquid to form a hardening paste. Alternatively, the calcium phosphate cement may be produced by solid-state acid-base reaction of crystalline calcium phosphate reactants to form crystalline calcium phosphate solids. Other methods of making calcium phosphate matrix are known in the art.

The calcium phosphate material may, for example, be crystalline hydroxyapatite (HA). Crystalline HA is described, for example, in U.S. Pat. Nos. Re. 33,221 and Re. 33,161 to Brown and Chow, both of which are herein incorporated by reference. The Brown and Chow Patents teach preparation of calcium phosphate remineralization compositions and of a finely crystalline, non-ceramic, gradually resorbable hydroxyapatite carrier material based on the same calcium phosphate composition. A similar calcium phosphate system, which consists of tetracalcium phosphate (TTCP) and monocalcium phosphate (MCP) or its monohydrate form (MCPM), is described by Constantz et al. in U.S. Pat. Nos. 5,053,212 and 5,129,905. In this embodiment, the calcium phosphate material is produced by solid-state acid-base reaction of crystalline calcium phosphate reactants to form crystalline hydroxyapatite solids.

Crystalline HA materials may be prepared such that they are flowable, moldable, and capable of hardening *in situ* (see U.S. Pat. No. 5,962,028 to Constantz). These HA materials (commonly referred to as carbonated hydroxyapatite) can be processed by combining the wet and dry reactants to provide a substantially uniform mixture, shaping the mixture as appropriate, and allowing the mixture to harden. Alternatively, precursor reaction mixtures can be administered to the surgical site and hardened and/or shaped *in situ.* During hardening, the mixture crystallizes into a solid and essentially monolithic apatitic structure.

The reactants will generally comprise a phosphoric acid source substantially free of unbound water, an alkali earth metal, particularly calcium, source, optionally crystalline nuclei, particularly hydroxyapatite or calcium phosphate crystals, calcium carbonate, and a physiologically acceptable lubricant, such as water, which may have various solutes. The dry ingredients may be pre-prepared as a mixture and subsequently combined with the liquid ingredients under conditions in which substantially uniform mixing occurs.

The phosphoric acid source may be any partially neutralized phosphoric acid, particularly up to and including complete neutralization of the first proton as in calcium phosphate monobasic. Alternatively or additionally, it can comprise orthophosphoric acid, possibly in a crystalline form, that is substantially free of uncombined water. Calcium sources will generally include counterions such as carbonate, phosphate or the like, particularly dual sources of calcium phosphate and phosphate such as tetracalcium phosphate or tricalcium phosphate, such as α/ß-tricalcium phosphate.

The various dry components may be combined prior to the addition of the wet components. Mixing combines the ingredients and can be used to regulate the extent of the inter-ingredient reactions. Any or all of the dry ingredients may be added prior to the initiation of mixing or prior to the completion of mechanical mixing. After mixing, the mixture is allowed to anneal while remaining quiescent, followed by an extended period of time during which the mixture hardens.

The resorption rate of the hardened CaP composition is adjusted by providing calcium phosphate phases that resorb at different speeds. For example, larger, less quickly resorbing particles, such as HA particles, can be embedded in fast resorbing phases such as brushite or amorphous calcium phosphate or vice versa. Preferably the size of certain particles and percentile of each phase should be chosen so that slower resorbing particles have a mean distance from each other of more than 50 µm, preferably more than 100 µm, and even more preferably more than 200 µm. The larger, slower resorbing particles may, in certain embodiments, additionally comprise a pore structure in the submicrometer range, which can serve as a "depository" for biologically active agents such as GDF-5 and/or GDF-3.

Such pores may comprise fast resorbing calcium phosphate material and thus mediate, on the one hand, a fast release of the biologically active agent(s), but on the other hand protect the biologically active agent(s) from the chemical reactions that take place during the hardening of the composition. The depository will allow an extended release of biologically active agent in the cement formed, while autologous bone formation rapidly proceeds throughout the surrounding fast resorbing material. Further avenues of influencing the resorption rate are pore structure, porosity, and size of pores of the hardened composition. These parameters can be used to influence the total amount of resorbing material as well as the surface/volume ratio, which also affects the resorption kinetics.

### Gas-Forming Agent

In certain embodiments, the compositions include a gas-forming agent, which will facilitate the process for producing a calcium phosphate matrix or scaffold material. The gas-forming agent may in particular be used to create pores in the hardened composition, in particular interconnecting pores for vascularization and cell ingrowth. In other embodiments, it will be helpful in producing a composition whose calcium phosphate material "self-granulates" and disperses into hardened macrogranules or macroparticles under physiological conditions (i.e., post-administration). The addition of a gas-forming agent to the calcium phosphate materials may alter the biological, chemical, and mechanical properties of the material and may enhance its therapeutic utility in certain applications. The gas-forming agent of the present invention may be any pharmaceutically acceptable substance which produces bubbling or liberation of a gas at physiological temperatures and/or pressures.

Methods for producing calcium phosphate materials for use with seeded compositions often suffer from limited injectability due to granule formation during production or preparation for administration in the syringe.

An added gas-forming agent may mitigate these drawbacks of the prior art. The gas-forming agent may be added to the other components of the composition (e.g., calcium phosphate material and any supplementary materials) to cause gas-foaming or -bubbling under specific conditions (i.e., physiological temperatures and/or pressures). The gas formation may assist in producing a desirable porosity and pore structure in the calcium phosphate material as discussed above upon injection or implantation *in vivo.*

The gas-forming agent may be a gas which is dissolved in the hydrated calcium phosphate material. The gas may be dissolved in the material under pressure, i.e., by subjecting the composite material to a pressurized atmosphere of the gas, but which is inert to the cementing reaction. The gas is then liberated upon exposure to physiological temperatures (i.e., upon injection or implantation), due to the decrease in gas solubility with increased temperature. Under these circumstances, the gas dissolution occurs only during hardening *in vivo,* and not prior to administration. By way of example only, suitable gases include hydrogen, carbon dioxide, air, nitrogen, helium, oxygen, and argon.

The gas-forming agent may be a volatile liquid which vaporizes at physiological temperatures. The gas-forming agent may also be a liquid that decomposes forming gas, such as H₂O₂.

The gas-forming agent may be a solid material which liberates gas upon dissolution. For example, sodium bicarbonate evolves carbon dioxide gas as it converts to an unstable carbonic acid intermediate, which subsequently evolves carbon dioxide and water.

### Bone-Forming Cells

The composition of the present invention is in many embodiments preferably cell-free and will only be populated with cells, in particular the patient's own bone-forming cells *in situ.* However, in certain embodiments, in order to optimize ossification, the calcium phosphate composition may be seeded with bone-forming cells, such as progenitor cells, stem cells, and/or osteoblasts. This is most easily accomplished by placing the calcium phosphate composition in contact with a source of the patient's own bone-forming cells. Such cells may be found in bone-associated tissue, blood or fluids, including exogenous fluids which have been in contact with bone or bone materials or regions, including the periosteum, cancellous bone or marrow.

When used in conjunction with devices such as screws and pins, the introduction of which into bone is accompanied by breach of the periosteum and/or bleeding, no further seeding is required. For plates, which oppose only cortical bone, induction of a periosteal lesion which will contact the device is generally recommended. In yet other embodiments, it will be useful to surgically prepare a seating within the bone by removing a portion of cortical bone at the implant site. Bone-forming cells harvested from the patient may be introduced into the graft to augment ossification. Use of non-autologous bone cells is also within the scope of the invention if the desired amount of bone regeneration occurs prior to host rejection of the bone-forming cells. Thus, cells or tissues obtained from primary sources, cell lines or cell banks may all be useful in certain embodiments. See, U.S. Pat. No. 6,132,463 to Lee et al., which is incorporated herein by reference.

### Supplementary Material

The composite material of the present invention may be prepared by combining the calcium phosphate material, biologically active agent and, optionally, a gas-forming agent, with a selected supplementary material. The calcium phosphate material may serve as the reinforcing material, the matrix or both. The calcium phosphate material in its initial hydrated form typically maintains a pH of about 6-7 and is therefore compatible with a wide range of additives without deleterious effect. The supplementary material is selected based upon its compatibility with calcium phosphate and the other components and its ability to impart properties (biological, chemical, or mechanical) to the composite, which are desirable for a particular therapeutic purpose. For example, the supplementary material may be selected to improve tensile strength and hardness, increase fracture toughness, provide imaging capability, and/or alter flow properties, and setting times of the calcium phosphate material. For example, the supplemental material may be added to provide for an elastic modulus of 100 to 1000 MPa in the hardened composition. In another example, the supplementary material may be designed to contain the calcium phosphate material at the desired site.

Supplementary material is added to the calcium phosphate composition in varying amounts and in a variety of physical forms, dependent upon the anticipated therapeutic use. For example, the supplementary material may be in the form of solid structures, such as sponges, meshes, films, fibers, gels, filaments, or particles, including macro- and nanoparticles. The supplementary material itself may be a composite. The supplemental material is a polymeric structure, in the form of a microscaffold. Such a microscaffold may be between 500 µm and 1 mm in size, and contain pores that prefably have a diameter of about 10 to about 1000 µm, preferably about 30 to about 500 µm, preferably about 100 to about 200 µm.

Microscaffolds with pores having diameters in the µm range rather than, for example, in the nm range further cell migration and provide access for unobstructed vascularization. These microscaffold pores can also be filled with CaP material. In certain preferred embodiments the microscaffolds are coated with a biocompatible material, preferably a nanocrystalline calcium phosphate. The microscaffold may be made of a variety of materials. In a preferred embodiment the microscaffolds are made from resilient material such as chitosan and/or a poly-lactic-acid/poly-e-caprolactone (PLA-PCL) copolymer. The resilience of these materials allows the microscaffold to have a size larger than the inner diameter of a typical injection needle, which generally is from 2 mm to 3 mm but may be up to 10 mm. The supplementary material also includes, in certain embodiments, hollow beads, which may provide more "pore-like" structures. In other embodiments, the supplemental material may also come in the form of non-hollow, nano- or macro particles.

Depending on the calcium phosphate material used, the pores have preferably a diameter of less than 5 mm, but preferably less than about 3 mm, less than about 2 mm, less than about 1 mm, less than about 0.5 mm or even less than about 0.25 mm, less than about 0.1 mm, less than about 0.05 mm, but generally not lower than 0.01 mm. The size of the pores is preferably selected to allow tissue ingrowth and GDF exit while containing the calcium phosphate material in the pouch. Generally, the pores will be chosen as small as possible so as to prevent leakage of the calcium phosphate material, since leaking calcium phosphate material could impinge upon nerves or blood vessels. However, at least one pore opening may be larger than the rest to allow the pouch to be filed. Preferably even this pore opening is less than 5 mm in diameter. However, its diameter will be depend on the needle, catheter or similar used to fill the pouch.

The size and density of the pores determine the ease or difficulty with which materials may pass through. For instance, very small pores (< 0.5 mm) generally would prohibit passage of all but the smallest particles and liquids. A combination of pores sizes and/or densities may allow for a "controlled" release of the GDF, i.e., a release of GDF over time. For example, two or more pore sizes could be combined to allow, e.g., for an initial peak in release of GDF, followed by a low, but constant release over, e.g., a period of at least one month, at least two months or preferably at least three months. In certain embodiments at least a fraction of the GDF remains in the pouch for, e.g., one year up to a lifetime, preferably one to ten years. As the person skilled in the art will appreciate, in certain applications, a shorter retention time may be preferable, e.g., about 1 year or less, while in other applications, a longer retention time may be preferable. Longer retention times allow in certain embodiments for low dosage administration regimes, such as from about 100 µg or less, up to 1 mg. The pore size and density may be controlled in the manufacturing process.

The disclosure comprises a supplementary material which may be a particulate or liquid additive or doping agent which is intimately mixed with the resorbable calcium phosphate. When intimately mixed with a calcium phosphate material, the supplementary material may interfere on a macroscopic level with the cementing reaction. This may occur with the supplementary material coating a percentage of the cement particles, allowing a weak cementing reaction to occur with the coated particles. Alternatively, the liquid or solid may cause physical separation between the reactive species resulting in focal areas of cement formation (or granules). The supplementary material may serve as a matrix for the calcium phosphate, which is embedded or dispersed within the matrix. Alternatively, the calcium phosphate may serve as a matrix for the supplementary material, which is dispersed therein. The supplementary material may be applied as a coating onto a calcium phosphate body, for example, as a post-fabrication coating to retard resorption time or otherwise affect the bioceramic material properties. Lastly, the supplementary material may be coated with the calcium phosphate composition.

The supplementary materials are preferably biocompatible, that is, there is no detrimental reaction induced by the material when introduced into the host. In many instances, it is desirable that the supplementary material also be bioresorbable. The supplementary material may have an affinity for calcium, phosphate or calcium phosphates which will enhance the strength of the calcium phosphate/ supplementary material interface. The affinity may be specific or mediated through non-specific ionic interactions. The supplementary material may be bioerodible, that is, susceptible to degradation over time, usually months (while a a "non-erodible" or "poorly erodible" material is, if at all, degraded over decades or at best, many years).

By way of example only, suitable bioerodible polymers for use as a matrix in the composition include, but are not limited to, collagen, glycogen, chitin, celluloses, starch, keratins, silk, nucleic acids, demineralized bone matrix, derivativized hyaluronic acid, polyanhydrides, polyorthoesters, polyglycolic acid, polylactic acid, and copolymers thereof. In particular, polyesters of [alpha] hydroxycarboxylic acids, such as poly(L-lactide) (PLLA), poly (D, L-lactide) (PDLLA), polyglycolide (PGA), poly (lactide-co-glycolide (PLGA), poly (D, L-lactideco-trimethylene carbonate), and polyhydroxybutyrate (PHB), and polyanhydrides, such as poly (anhydride-co-imide) and co-polymers thereof are known to bioerode and are suitable for use in the present invention. In addition, bioactive glass compositions, such as compositions including SiO₂, Na₂O, CaO, P₂O₅, A₁₂O₃ and/or CaF₂, may be used in combination with the calcium phosphate composition of the invention. Other useful bioerodible polymers may include polysaccharides, peptides, and fatty acids.

Bioerodible polymers are advantageously used in the preparation of bioresorbable hardware, such as but not limited to intermedulary nails, pins, screws, plates, and anchors for implantation at a bone site. The bioresorbable fiber may be in the form of whiskers which interact with calcium phosphates according to the principles of composite design and fabrication known in the art. Such hardware may be formed by pressing a powder particulate mixture of the calcium phosphate and polymer. Alternatively, the calcium phosphate matrix may be reinforced with PLLA fibers, using PLLA fibers similar to those described by Tormala et al., which is incorporated herein by reference, for the fabrication of biodegradable self-reinforcing composites (Clip. Mater. 10:29-34 (1992)).

Bioresorbable polymers may also be used in the preparation of bone glues or putties for use in load-bearing situations. Supplementary materials may be added to the composite to increase compressibility and load-bearing properties of the bone glue. In particular, carbon fibers or other reinforcing fibers may be added to the composite. In the production of fiber-reinforced bone substitute glues, it may be advantageous to plasma-etch the fibers to improve the quality and strength of the calcium phosphate/fiber interface. Calcium phosphate may also be hardened at 37 °C, pulverized or otherwise fragmented, and mixed with known binders such as bone glues, cements, fillers, plasters, epoxies, other calcium phosphates, or gels such as, but not limited to, calcium sulfate, alginate, collagen, or commercially available products such as Endobone (Merck), Hapset (Lifecore Biomedical), SRS(R) (Norian), Bonesource(R) (Leibinger), Collograft(R) (Zimmer), Osteograf(R) (CereMed), and Simplex(R) (Howmedica). For applications in which hardened calcium phosphate will be dispersed within the binder substance, most often the binder will be prepared by methods known to the art and mixed with the particulate calcium phosphate in approximately equal volumes, although actual proportions will be varied in ways known to the art to produce compositions of desired consistency, workability, and adherence.

In yet another disclosure, braided sutures, typically prepared from polyester, maybe coated with the calcium phosphate composition of the invention, to improve their biocompatibility. Coated sutures may be prepared by dipping the suture into a slurry containing the calcium phosphate material. The affinity of the suture for the calcium phosphate coating may be improved by surface treating either the suture, the calcium phosphate particle or both. Surface treatments include plasma- etching and/or chemical-grafting.

A composite may be provided comprising the calcium phosphate material and a non-resorbable or poorly resorbable material. Suitable non-erodible or poorly erodible materials include dextrans, polyethylene, polymethylmethacrylate (PMMA), carbon fibers, polyvinyl alcohol (PVA), poly (ethylene terephthalate) polyamide, bioglasses, and those compounds listed previously for use in bone glues or putties.

Another use may be to permanently imbed useful objects, such as a pin or reinforcing mesh, into bone itself. The object serves as an anchor for the stable attachment to natural bone. This is particularly useful in the attachment of ligaments and tendons to bone. Objects comprising bioresorbable and ossifying calcium phosphate and a suitable non-resorbable hardware may be placed into a bone and further secured with additional calcium phosphate material or composite material in a bone glue formulation. The hardware then becomes embedded into the bone following reossification of the calcium phosphate material.

In yet another disclosure of the invention, a composition is prepared by blending the calcium phosphate material with an additive which alters the resorption properties, setting time and/or flow characteristics of the composition. For example, silicone oil or other lubricating polymers or liquids may be added to the composition to improve the flow characteristics of the composition for delivery to the host by syringe. The lubricant is preferably biocompatible and capable of rapid leaching from the bone-substitute material composite following solidification of the calcium phosphate composition *in vivo.* Suitable lubricants include, by way of example only, polymer waxes, lipids, and fatty acids. Lubricants may be used in a concentration of about 0.1 to about 30 wt %.

In yet another disclosure of the invention, a cement, ergo a hardened composition, is prepared by admixing to the composition a liquid, which will, together with the calcium phosphate material form a hardening solid phase, for example, partially neutralized phosphoric acid and calcium carbonate. During this reation CO₂ may form. Alternatively, monocalciumphosphate anhydrite, α/ß-tricalcium phosphate, and water may be combined and pores may be introduced via the mixing process.

Pores having a diameter of more than 100 µm can be achieved by the production of gas bubbles, e.g., by decomposing H₂O₂ into water and oxygen. Here H₂O₂ may be added to the composition at a concentration of 4% and at a (molar) ratio of 10:3. The size of the resulting bubbles, and thus the size and length of the pore canals which result from the escaping bubbles, can be adjusted by the concentration of H₂O₂ und the viscosity of the mixture.

Calcium phosphate comprising nanostructures can be produced, e.g., via original compositions comprising powders in the nanometer range. The agglomeration of these nanopowders and the resulting nanopores can be regulated by the production process of the powders (e.g., "pulse combustion", IBUtec, Weimar, Germany). Larger pores (but preferably smaller than 1 µm) can be provided by producing coarser powders from these agglomerates. Here, the particular size of the powder can also be adjusted via the production process. By means of a thermal treatment the composition of the phases can be adjusted, e.g., by a transformation of hydroxylapatite to α/ß-tricalcium phosphate. A thermal treatment in which sintering temperatures are exceeded will result in spherical pores depending on the parameters of the thermal treatment.

The combination of prestructured, in particular nanoporous, powders, and gas-forming agents, may result in materials that combine nanoporosity and macroporosity.

The following examples detail presently preferred embodiments of the invention. Numerous modifications and variations in practice thereof are expected to occur to those skilled in the art upon consideration of these descriptions. Those modifications and variations are believed to be encompassed within the claims appended hereto. These examples do not in any way limit the invention.

### EXAMPLES

### Reference-

### Example 1:

Forty-eight (48) aged, osteoporotic sheep were subdivided into 8 groups and treated with different dosages of non-glycosylated recombinant human GDF-5 (Fig. 1 (C)) (Hortschansky et al., Hans-Knöll Institute, Jena, Germany) via a fusion protein precursor (Fig. 1 (B)) from the nucleotide sequence shown in Fig. 1 (A) and non-glycosylated recombinant human BMP-2 (Hortschansky et al., Hans-Knöll Institute, Jena). Groups 1, 3, 5, and 7 were treated with a minimal dosage of a composition comprising hydroxyapatite (HA) (CAMCERAM, HA protein coating powder, CAM Implants, B.V. Leiden, The Netherlands)) and rhBMP-2 and rhGDF-5, respectively, while groups 2, 4, 6, and 8 were treated with the maximal dosages of these compositions. A control (vertebral body not treated) and a HA only group in which the vertebral body was only treated with HA, were also included in the study. The experimental design is depicted in Table 2, which indicates that observations were made short-term (three months) and long-term (nine months). Five (5) mg HA particles were used per 100 µl osteogenic factor containing serum.

**Table 2**

| **Growth Factor** | **Dose [µg]** | **Time Period [months]** |
|---|---|---|
| rhBMP-2 | 1 (minimal) | 3 (short) |
| | | 9 (long) |
| | 10 (maximal) | 3 |
| | | 9 |
| rhGDF-5 | 5 (minimal) | 3 |
| | | 9 |
| | 50 (maximal) | 3 |
| | | 9 |

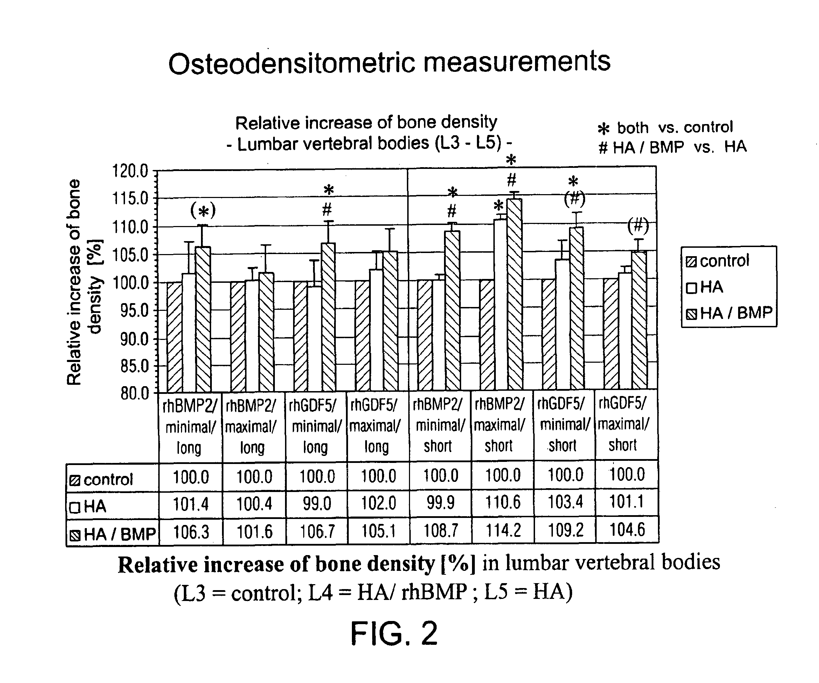

The lumbar vertebral body of the animals was injected with the compositions comprising rh-BMP-2 or rhGDF-5. After three months a significant increase in the bone mineral density (BMD) could be observed and was still present after nine months. The BMD was measured in the central spongious part of the vertebral body excluding the boney cortical area using the DEXA QDR 4500 Elite (HOLOGIC) as a BMD measurement device. The results are shown in Fig. 2. GDF-5, which previous data showed to exhibit only about 20% of the activity of BMP-2 and thus require a five times higher dosages to achieve results equivalent to BMP-2 was found to result in about the same, and even slightly higher bone density, at half the concentration of BMP-2 (5 µg GDF-5 vs. 10 µg BMP-2). Thus, GDF-5 when attached to CaP carriers opens an avenue of providing osteogenic factors at a lower dosage than the currently available BMP-2 and BMP-7 compositions. The study also included measurement of the compression strength in which extracted 10 x 15 mm cylinders from prepared and treated vertebral bodies (control, HA, and "HA-BMP" (HA-BMP-2/ HA-GDF-5) were subjected to compression.

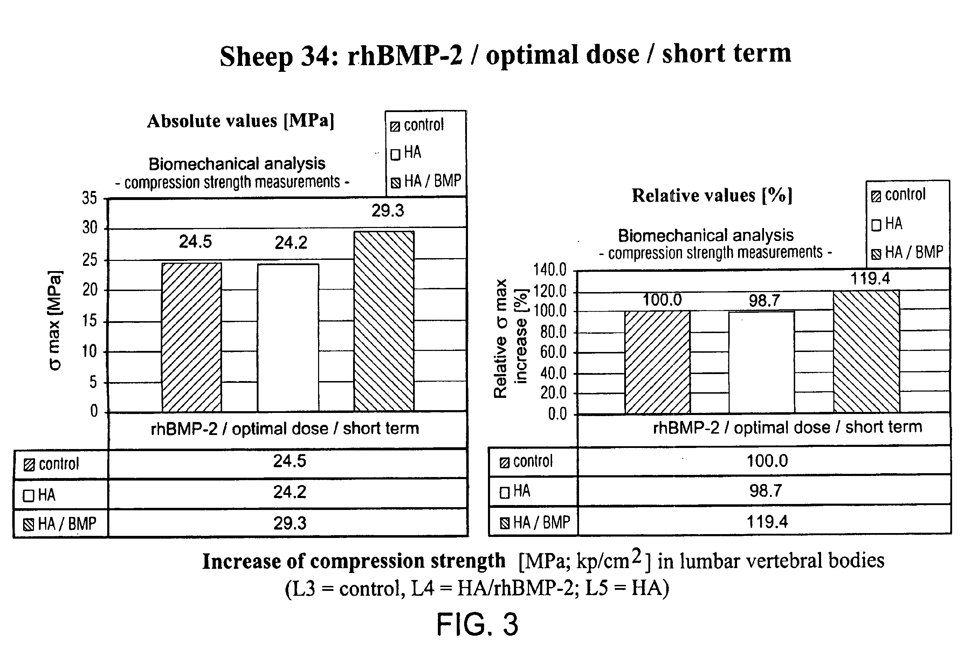

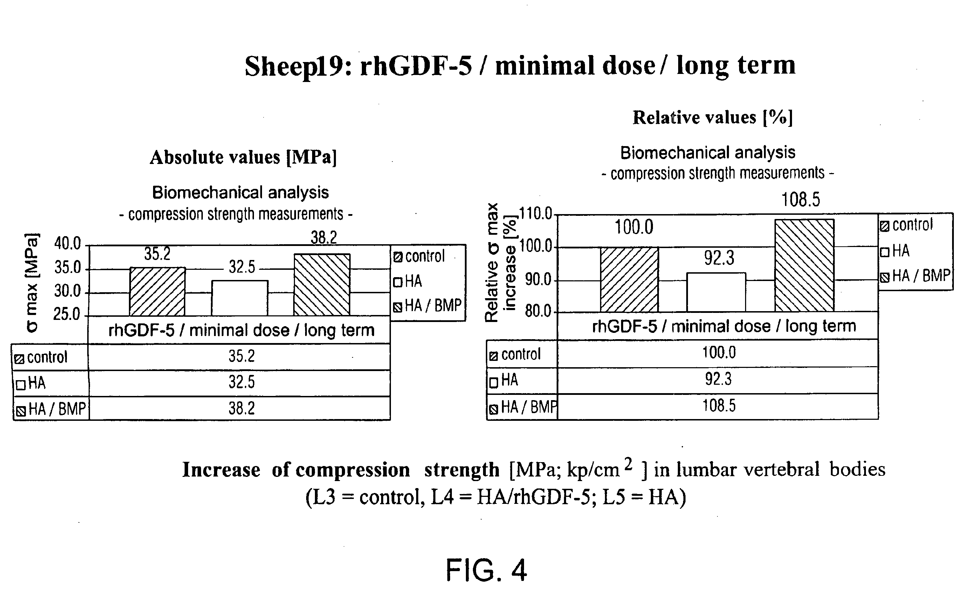

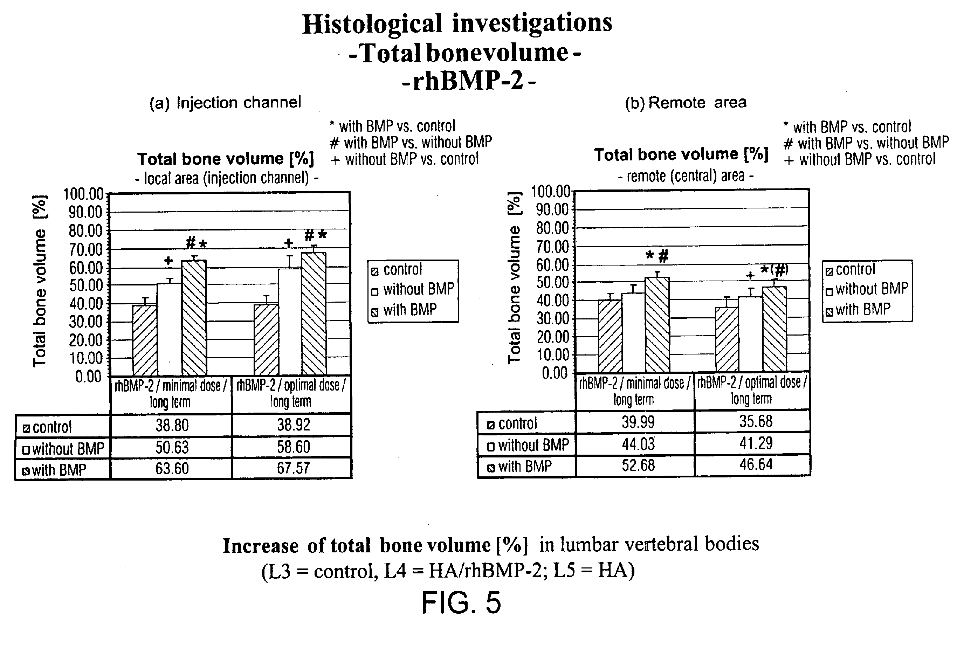

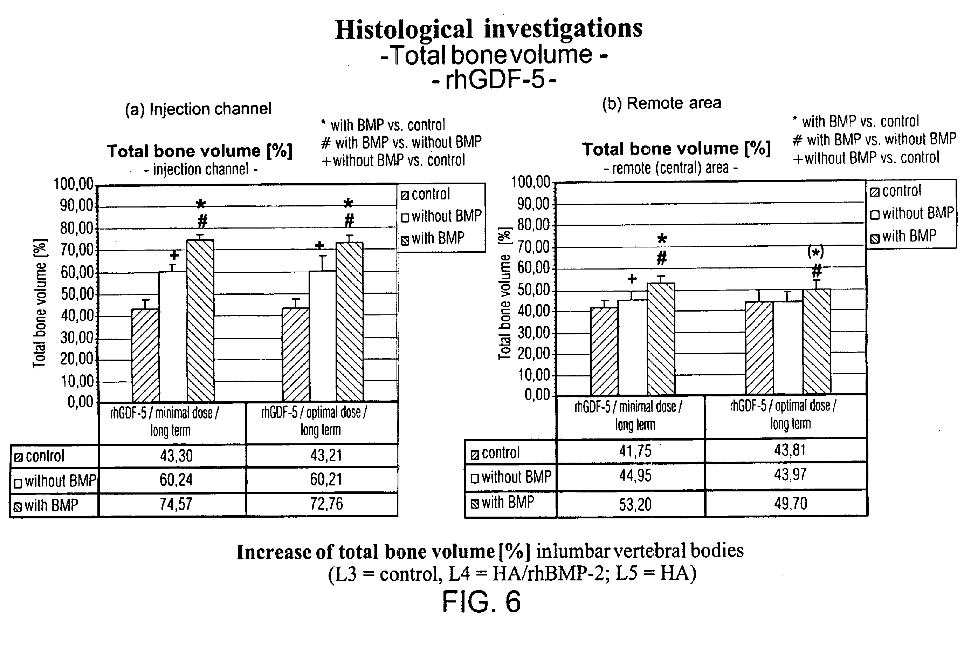

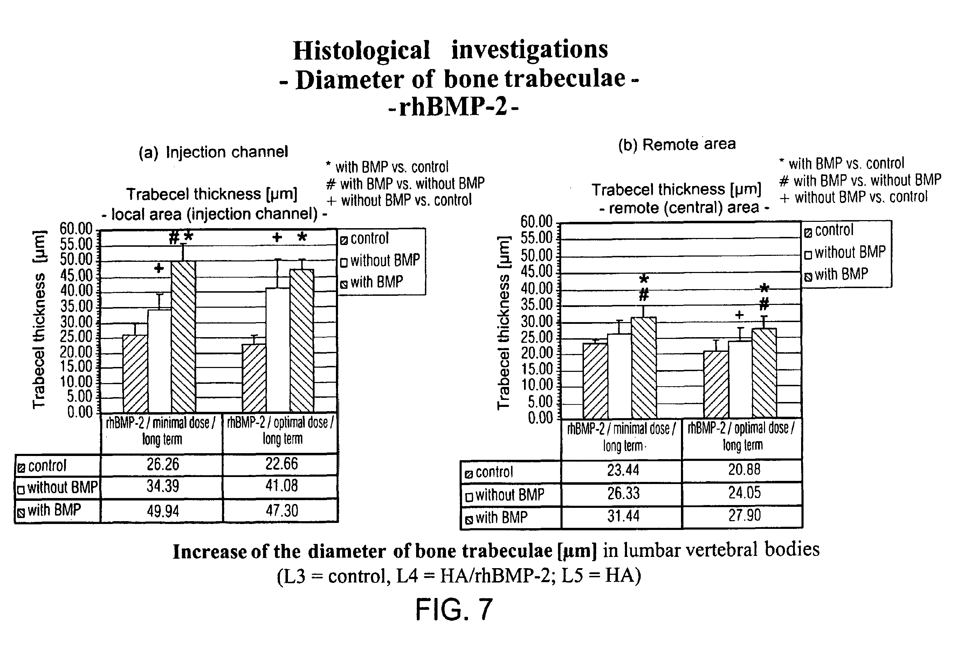

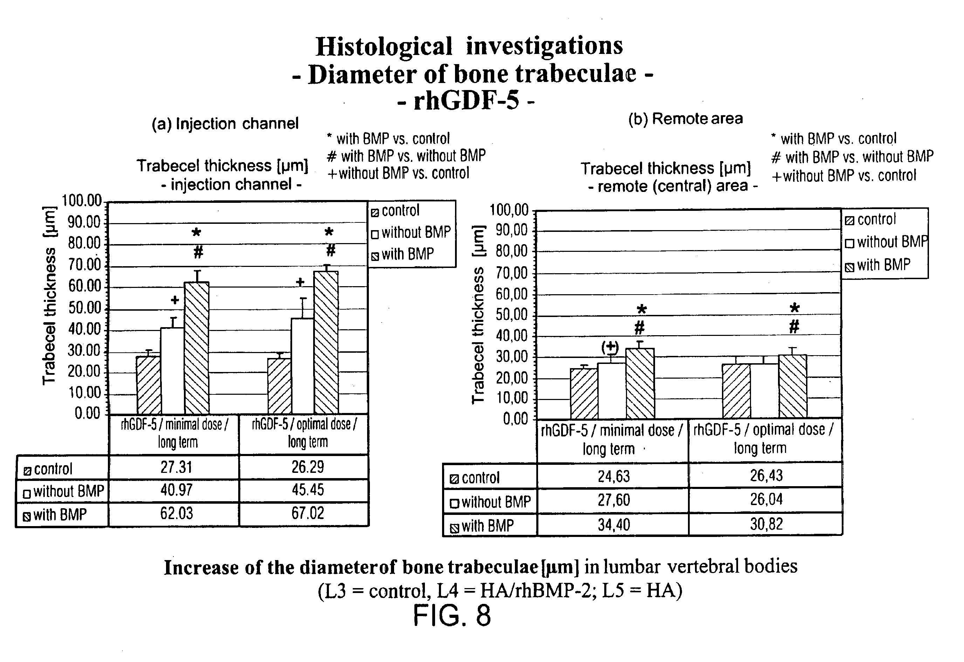

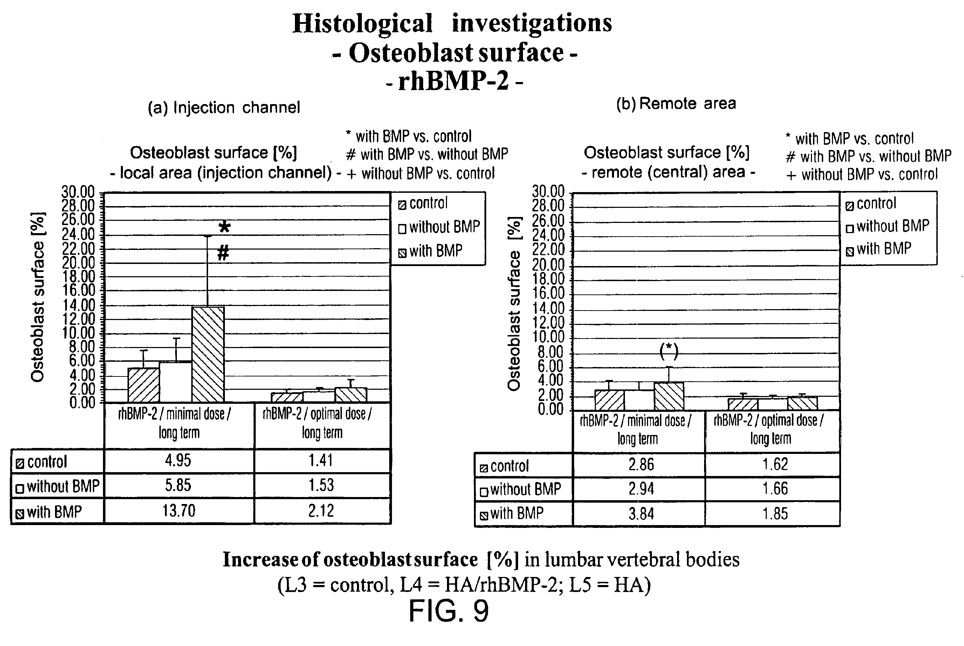

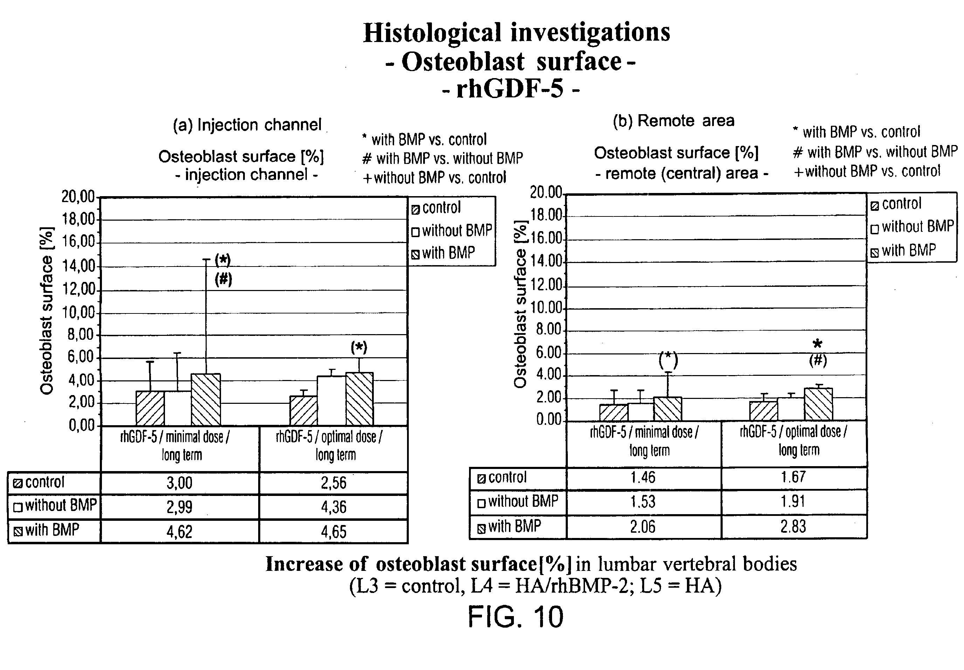

Figs. 3 and 4 show that both in absolute terms, as well as in relative terms, the HA together with the "minimal" dose of GDF-5 (5 µg) compares well with the higher, "maximal" dose of BMP-2 (10 µg) in these compression studies. Figs. 5 and 6 also show that the "minimal" dose of GDF-5 (5 µg) compares well with the "maximal" dose of BMP-2 (10 µg) in terms of total bone volume. In both cases, an about 30% increase at the injection channel could be shown as well as an about 10% increase in the remote central area. The remote area can have a distance of more than 5 mm and up to 10 mm from the injection channel, showing that the composition of the present invention displayed activity beyond an area that the person skilled in the art would have expected to be reached by simple diffusion. Figs. 7 and 8 also show that the "minimal" dose of GDF-5 (5 µg) compares well with the "maximal" dose of BMP-2 (10 µg) in terms of their effect on the diameter of bone trabeculae. In both cases, an about 30% increase at the injection channel was observed as well as an about 10% increase in the remote central area. Finally, Figs. 9 and 10 show that the "minimal" dose of GDF-5 (5 µg) compares well with the "maximal" dose of BMP-2 (10 µg) in terms of osteoblast surface. In both cases, an increase at the injection channel was observed as well as some increase in the remote central area.

### Example 2:

A CaP-GDF composition comprising calciumhydrogenphosphate anhydride, a microscaffold coated with nanocrystalline calciumhydrogenphoshate anhydride, GDF-5 at a total concentration of 100 µg is provided. The composition is admixed with a buffer solution to reach a suitable viscosity for injection and is injected into a fractured vertebral body of a patient with a 9 gauge injection needle having an inner diameter of 2.2 mm. The injected CaP-GDF composition hardens *in situ.* During the hardening process pores are formed. The pores formed have an average diameter of less than 100 µm. The compression strength of the hardened CaP-GDF composition is less than 100 MPa and has an elastic modulus of more than 1000 MPa. GDF is released into the fractured vertebral body and induces and/or furthers osteogenesis in the vertebral body despite the pathological reduction of osteogenesis in the patient. New bone initially forms in the pores of the hardened composition and stepwise replaces parts of the bioresorbable CaP material. After one year 70% of the CaP material is replaced by newly-formed bone.

### Example 3:

Four to six weeks after the procedure in Example 2 has been completed, a composition as described in Example 2 is injected into vertebral bodies adjacent to the fractured vertebral body of the patient in Example 2. Upon injection, bone formation is also induced and/or furthered in those adjacent vertebral bodies to increase their compression strength. This preventive treatment of the adjacent vertebral bodies reduces the risk of subsequent fractures.

### Example 4:

The compositions according to the invention may comprise several calcium phosphate materials that are resorbed at different speeds, that is have, upon hardening, differing resorption rates, depending on the purpose of application.

A formulation suitable for use in spinal column surgery may contain (as final composition) 55% dicalcium phosphate dihydrate (DCPD) and 45% ß-tricalcium phosphate (ß-TCP), wherein in the DCPD portion the average pore size is less than 5 µm and in the ß-TCP portion the average pore size is between 2 and 50 µm, and wherein pores with a size of about 1 µm are present in relatively large numbers (proportions) but pores with a size of about 200 µm are present in relatively small numbers (proportions). That is pores with a size of about 1 µm numerically predominate over pores with a size of about 200 µm. The ß-TCP-phase of this calcium-phosphate-cement has a medium resorption rate (solubility at 37°C: -log (Kₛ) = 29.5) whereas the DCPD-phase of that cement has a significantly higher (about 5 times higher) resorption rate than the ß-TCP phase. The final cement is mainly resorbed *in vivo* after 6 to 12 months.

A formulation suitable for use in the therapy of bone traumata of relatively young patients should have an *in vivo* resorption period of about 3 months. Such formulation may comprise a calcium phosphate material containing none or nearly none hydroxylapatite (low resorption rate), but 40-100%, preferably 50-75%, more preferably 50-60% dicalcium phosphate dihydrate (DCPD) (relatively high resorption rate) and 30-100%, preferably 40-60%, more preferably 40-50% ß-tricalcium phosphate (ß-TCP) (medium resorption rate).

A formulation suitable for use in the therapy of middle-age patients with osteoporosis should have an *in vivo* resorption period of about 3 to 24 months. Such formulation may comprise a calcium phosphate material containing 1 to 15% hydroxylapatite (low resorption rate), 40-99%, preferably 50-75%, more preferably 50-60% dicalcium phosphate dihydrate (DCPD) (relatively high resorption rate) and 30-99%, preferably 40-60%, more preferably 40-50% ß-Tricalcium phosphate (ß-TCP) (medium resorption rate).

A formulation suitable for use in the therapy of old patients with osteoporosis should have an *in vivo* resorption period of more than 24 months. Such formulation may comprise a calcium phosphate material containing 16 to 100%, preferably 40-100% hydroxylapatite, 40-84%, preferably 50-75%, more preferably 50-60% dicalcium phosphate dihydrate (DCPD) and 30-84%, preferably 40-60%, more preferably 40-50% ß-tricalcium phosphate (ß-TCP), wherein the *in vivo* resorption period of said portion is more than 24 months.

In general it may be preferable that the bioresorbable calcium phosphate material of the composition according to the invention comprises
- a first component/portion (I) containing a calcium phosphate having a solubility given as -log(Kₛ) at 37 °C of between about 1 and 10, and/or
- a second component/portion (II) containing a calcium phosphate having a solubility given as -log(Kₛ) at 37 °C of between about 10 and about 50, and/or
- a third component/portion (III) containing a calcium phosphate having a solubility given as -log(Kₛ) at 37 °C of more than 50, and wherein
- either (a) portion (I) is about 40-100%, preferably 50-75%, more preferably 50-60%, and portion (II) is 30-100%, preferably 40-60%, more preferably 40-50%, and portion (III) is about 0% to 1%, whereby the resulting composition is suitable for purposes of repairing, regenerating and/or augmenting bone tissue of patients in the age of less or equal to about 30 years;
- or (b) portion (I) is 40-99%, preferably 50-75%, more preferably 50-60%, and portion (II) is 30-99%, preferably 40-60%, more preferably 40-50% and portion (III) is 1 to 15%, whereby the resulting composition is suitable for purposes of repairing, regenerating and/or augmenting bone tissue of patients older than 30 years;
- or (c) portion (I) is 40-84%, preferably 50-75%, more preferably 50-60%, and portion (II) is 30-84%, preferably 40-60%, more preferably 40-50%, and portion (III) is 16-100%, preferably 40-100%, and whereby resulting composition is suitable for purposes of repairing, regenerating and/or augmenting bone tissue of patients with an osteoporotic condition.

### SEQUENCE LISTING

<110> venbrocks, Rudolf A. Jandt, Klaus D. Kinne, Raimund W. Bossert, Joerg Hortschansky, Peter Schmuck, Klaus
<120> CALCIUM PHOSPATE BASED DELIVERY OF GROWTH AND DIFFERENTIATION FACTORS TO COMPROMISED BONE
<130> DPG153/0B/WO
<150> US 60/970,258
   <151> 2007-09-05
<150> EP 07022543
   <151> 2007-11-21
<150> US 11/928,889
   <151> 2007-10-30
<160> 4
<170> PatentIn version 3.3
<210> 1
   <211> 9
   <212> PRT
   <213> Artificial
<220>
   <223> Partial GDF-5 sequence
<400> 1
   Met Asn Ser Met Asp Pro Glu Ser Thr
   1               5
<210> 2
   <211> 1557
   <212> DNA
   <213> Artificial
<220>
   <223> Coding sequence for fusion protein of maltose-binding protein (MBP) and GDF-5
<400> 2
   atgaaaatcg aagaaggtaa actggtaatc tggattaacg gcgataaagg ctataacggt 60
   ctcgctgaag tcggtaagaa attcgagaaa gataccggaa ttaaagtcac cgttgagcat 120
   ccggataaac tggaagagaa attcccacag gttgcggcaa ctggcgatgg ccctgacatt 180
   atcttctggg cacacgaccg ctttggtggc tacgctcaat ctggcctgtt ggctgaaatc 240
   accccggaca aagcgttcca ggacaagctg tatccgttta cctgggatgc cgtacgttac 300
   aacggcaagc tgattgctta cccgatcgct gttgaagcgt tatcgctgat ttataacaaa 360
   gatctgctgc cgaacccgcc aaaaacctgg gaagagatcc cggcgctgga taaagaactg 420
   aaagcgaaag gtaagagcgc gctgatgttc aacctgcaag aaccgtactt cacctggccg 480
   ctgattgctg ctgacggggg ttatgcgttc aagtatgaaa acggcaagta cgacattaaa 540
   gacgtgggcg tggataacgc tggcgcgaaa gcgggtctga ccttcctggt tgacctgatt 600
   aaaaacaaac acatgaatgc agacaccgat tactccatcg cagaagctgc ctttaataaa 660
   ggcgaaacag cgatgaccat caacggcccg tgggcatggt ccaacatcga caccagcaaa 720
   gtgaattatg gtgtaacggt actgccgacc ttcaagggtc aaccatccaa accgttcgtt 780
   ggcgtgctga gcgcaggtat taacgccgcc agtccgaaca aagagctggc aaaagagttc 840
   ctcgaaaact atctgctgac tgatgaaggt ctggaagcgg ttaataaaga caaaccgctg 900
   ggtgccgtag cgctgaagtc ttacgaggaa gagttggcga aagatccacg tattgccgcc 960
   accatggaaa acgcccagaa aggtgaaatc atgccgaaca tcccgcagat gtccgctttc 1020
   tggtatgccg tgcgtactgc ggtgatcaac gccgccagcg gtcgtcagac tgtcgatgaa 1080
   gccctgaaag acgcgcagac taattcgagc tcgaacaaca acaacaataa caataacaac 1140
   aacctcgggc atcaccatca ccatcatgga tccgagaacc tgtacttcca gggtgcccca 1200
   ctggccactc gccagggcaa gcgacccagc aagaacctta aggctcgctg cagtcggaag 1260
   gcactgcatg tcaacttcaa ggacatgggc tgggacgact ggatcatcgc accccttgag 1320
   tacgaggctt tccactgcga ggggctgtgc gagttcccat tgcgctccca cctggagccc 1380
   acgaatcatg cagtcatcca gaccctgatg aactccatgg accccgagtc cacaccaccc 1440
   acctgctgtg tgcccacgcg gctgagtccc atcagcatcc tcttcattga ctctgccaac 1500
   aacgtggtgt ataagcagta tgaggacatg gtcgtggagt cgtgtggctg caggtag 1557
<210> 3
   <211> 518
   <212> PRT
   <213> Artificial
<220>
   <223> Fusion protein: Maltose-binding protein (MBP) and GDF-5
<400> 3
   Met Lys Ile Glu Glu Gly Lys Leu Val Ile Trp Ile Asn Gly Asp Lys
   1               5                   10                  15
   Gly Tyr Asn Gly Leu Ala Glu Val Gly Lys Lys Phe Glu Lys Asp Thr
               20                  25                  30
   Gly Ile Lys Val Thr Val Glu His Pro Asp Lys Leu Glu Glu Lys Phe
           35                  40                  45
   Pro Gln Val Ala Ala Thr Gly Asp Gly Pro Asp Ile Ile Phe Trp Ala
       50                  55                  60
   His Asp Arg Phe Gly Gly Tyr Ala Gln Ser Gly Leu Leu Ala Glu Ile
   65                  70                  75                  80
   Thr Pro Asp Lys Ala Phe Gln Asp Lys Leu Tyr Pro Phe Thr Trp Asp
                   85                  90                  95
   Ala Val Arg Tyr Asn Gly Lys Leu Ile Ala Tyr Pro Ile Ala Val Glu
   100 105 110
   Ala Leu Ser Leu Ile Tyr Asn Lys Asp Leu Leu Pro Asn Pro Pro Lys
   115 120 125
   Thr Trp Glu Glu Ile Pro Ala Leu Asp Lys Glu Leu Lys Ala Lys Gly
   130 135 140
   Lys Ser Ala Leu Met Phe Asn Leu Gln Glu Pro Tyr Phe Thr Trp Pro
   145 150 155 160
   Leu Ile Ala Ala Asp Gly Gly Tyr Ala Phe Lys Tyr Glu Asn Gly Lys
   165 170 175
   Tyr Asp Ile Lys Asp Val Gly Val Asp Asn Ala Gly Ala Lys Ala Gly
   180 185 190
   Leu Thr Phe Leu Val Asp Leu Ile Lys Asn Lys His Met Asn Ala Asp
   195 200 205
   Thr Asp Tyr Ser Ile Ala Glu Ala Ala Phe Asn Lys Gly Glu Thr Ala
   210 215 220
   Met Thr Ile Asn Gly Pro Trp Ala Trp Ser Asn Ile Asp Thr Ser Lys
   225 230 235 240
   Val Asn Tyr Gly Val Thr Val Leu Pro Thr Phe Lys Gly Gln Pro Ser
   245 250 255
   Lys Pro Phe Val Gly Val Leu Ser Ala Gly Ile Asn Ala Ala Ser Pro
   260 265 270
   Asn Lys Glu Leu Ala Lys Glu Phe Leu Glu Asn Tyr Leu Leu Thr Asp
   275 280 285
   Glu Gly Leu Glu Ala Val Asn Lys Asp Lys Pro Leu Gly Ala Val Ala
   290 295 300
   Leu Lys Ser Tyr Glu Glu Glu Leu Ala Lys Asp Pro Arg Ile Ala Ala
   305 310 315 320
   Thr Met Glu Asn Ala Gln Lys Gly Glu Ile Met Pro Asn Ile Pro Gln
   325 330 335
   Met Ser Ala Phe Trp Tyr Ala Val Arg Thr Ala Val Ile Asn Ala Ala
   340 345 350
   Ser Gly Arg Gln Thr Val Asp Glu Ala Leu Lys Asp Ala Gln Thr Asn
   355 360 365
   Ser Ser Ser Asn Asn Asn Asn Asn Asn Asn Asn Asn Asn Leu Gly His
   370 375 380
   His His His His His Gly Ser Glu Asn Leu Tyr Phe Gln Gly Ala Pro
   385 390 395 400
   Leu Ala Thr Arg Gln Gly Lys Arg Pro Ser Lys Asn Leu Lys Ala Arg
   405 410 415
   Cys Ser Arg Lys Ala Leu His Val Asn Phe Lys Asp Met Gly Trp Asp
   420 425 430
   Asp Trp Ile Ile Ala Pro Leu Glu Tyr Glu Ala Phe His Cys Glu Gly
   435 440 445
   Leu Cys Glu Phe Pro Leu Arg Ser His Leu Glu Pro Thr Asn His Ala
   450 455 460
   Val Ile Gln Thr Leu Met Asn Ser Met Asp Pro Glu Ser Thr Pro Pro
   465 470 475 480
   Thr Cys Cys Val Pro Thr Arg Leu Ser Pro Ile Ser Ile Leu Phe Ile
   485 490 495
   Asp Ser Ala Asn Asn Val Val Tyr Lys Gln Tyr Glu Asp Met Val Val
   500 505 510
   Glu Ser Cys Gly Cys Arg
   515
<210> 4
   <211> 121
   <212> PRT
   <213> Homo sapiens
<400> 4

Individual Applicant
   -------------------
   Streets :
   City :
   State :
   Country :
   PostalCode :
   PhoneNumber :
   FaxNumber :
   EmailAddress :
<110> LastName : venbrocks
   <110> FirstName : Rudolf A.
   <110> MiddleInitial :
   <110> Suffix :
Individual Applicant --------------------
   Street :
   City :
   State :
   Country :
   PostalCode :
   PhoneNumber :
   FaxNumber :
   EmailAddress :
<110> LastName : Jandt
   <110> FirstName : Klaus D.
   <110> MiddleInitial :
   <110> Suffix :
Individual Applicant
   -------------------- Street :
   City :
   State :
   Country :
   PostalCode :
   PhoneNumber :
   FaxNumber :
   EmailAddress :
<110> LastName : Kinne
   <110> FirstName : Raimund W.
   <110> MiddleInitial :
   <110> Suffix :
Individual Applicant
   -------------------
   Street :
   City :
   State :
   Country :
   PostalCode :
   PhoneNumber :
   FaxNumber :
   EmailAddress :
<110> LastName : Bossert
   <110> FirstName : Joerg
   <110> MiddleInitial :
   <110> Suffix :
Individual Applicant
   --------------------
   Street :
   City :
   State :
   Country :
   PostalCode :
   PhoneNumber :
   FaxNumber :
   EmailAddress :
<110> LastName : Hortschansky
   <110> FirstName : Peter
   <110> MiddleInitial :
   <110> Suffix :
Individual Applicant
   --------------------
   Street :
   City
   State :
   Country :
   PostalCode :
   PhoneNumber :
   FaxNumber :
   EmailAddress :
<110> LastName : Schmuck
   <110> FirstName : Klaus
   <110> MiddleInitial :
   <110> Suffix :
Application Project
   -------------------
<120> Title : CALCIUM PHOSPATE BASED DELIVERY OF GROWTH AND DIFFERENTIATION FACTORS TO COMPROMISED BONE
   <130> AppFileReference : DPG153/0B/WO
   <140> CurrentAppNumber :
   <141> CurrentFilingDate : _-_-_
Earlier Applications --------------------
<150> PriorAppNumber : US 60/970,258
   <151> PriorFilingDate : 2007-09-05
Earlier Applications
   --------------------
<150> PriorAppNumber : EP 07022543
   <151> PriorFilingDate : 2007-11-21
Earlier Applications --------------------
<150> PriorAppNumber : US 11/928,889
   <151> PriorFilingDate : 2007-10-30
Sequence
   <213> OrganismName : Artificial
   <400> PreSequenceString :
   MNSMDPEST 9
   <212> Type : PRT
   <211> Length : 9
   SequenceName : 1
   SequenceDescription :
Sequence
   <213> OrganismName : Artificial
   <400> PreSequenceString :
<212> Type : DNA
<211> Length : 1557 SequenceName : 2 SequenceDescription :
Sequence
   --------
<213> OrganismName : Artificial
   <400> PreSequenceString :
<212> Type : PRT
<211> Length : 518
   SequenceName : 3
   SequenceDescription :
Sequence
   --------
<213> OrganismName : Homo sapiens
   <400> PreSequenceString : <212> Type : PRT
   <211> Length : 121
   SequenceName : 4
   SequenceDescription :

## Claims

1. A composition for repairing, regenerating and/or augmenting bone tissue wherein said composition delivers recombinant growth and differentiation factor(s) and comprises a bioresorbable calcium phosphate material and recombinant GDF-5 and/or GDF-3 as biologically active agent, **characterized in that**:
i) the composition further comprises an aqueous liquid and is injectable, whereby after injection it undergoes conversion to a hardened calcium phosphate (cement),
ii) the bioresorbable calcium phosphate material comprises at least two calcium phosphate species having different resorption rates and upon hardening resulting in a calcium phosphate cement, that contains pores and that comprises phases that resorb at different rates,
iii) the composition further comprises a bioerodible polymeric structure wherein said polymeric structure is/are microscaffold(s) having a porous structure.

2. The composition according to claim 1, wherein the calcium phosphate material is selected from the group of calcium phosphates (calcium phosphate species) consisting of monocalciumphosphate anhydrite, calciumhydrogenphosphate dihydrate, dicalciumphosphate dihydrate, brushite, calciumhydrogenphosphate anhydrite, hydroxyapatite, α-tricalcium phosphate, ß-tricalcium phosphate, fluorapatite, and a combination thereof.

3. The composition of claim 1 or 2, wherein
- a first portion (I) contains a calcium phosphate having a solubility given as -log(Kₛ) at 37 °C of between 1 and 10, and/or
- a second portion (II) contains a calcium phosphate having a solubility given as -log(Kₛ) at 37 °C of between 10 and 50, and/or
- a third portion (III) contains a calcium phosphate having a solubility given as -log(Kₛ) at 37 °C of more than 50, and wherein
- either (a) portion (I) is 40-100%, preferably 50-75%, more preferably 50-60%, and portion (II) is 30-100%, preferably 40-60%, more preferably 40-50%, and portion (III) is 0% to 1%, whereby the resulting composition is suitable for purposes of repairing, regenerating and/or augmenting bone tissue of patients in the age of less or equal to 30 years;
- or (b) portion (I) is 40-99%, preferably 50-75%, more preferably 50-60%, and portion (II) is 30-99%, preferably 40-60%, more preferably 40-50% and portion (III) is 1 to 15%, whereby the resulting composition is suitable for purposes of repairing, regenerating and/or augmenting bone tissue of patients older than 30 years;
- or (c) portion (I) is 40-84%, preferably 50-75%, more preferably 50-60%, and portion (II) is 30-84%, preferably 40-60%, more preferably 40-50%, and portion (III) is 16-100%, preferably 40-100%, and whereby the resulting composition is suitable for purposes of repairing, regenerating and/or augmenting bone tissue of patients with an osteoporotic condition.

4. The composition of one of claims 1 to 3, wherein, upon hardening, a first phase resorbs at first rate and optionally comprises pores having an average diameter of less than one µm, preferably wherein said calcium phosphate material comprises, upon hardening, interconnected pores, and a second phase resorbs at a second rate which exceeds that of said first rate.

5. The composition of claim 4, wherein said pores comprise parts of said second phase and said first phase optionally essentially consists of particles.

6. The composition according to one of claims 1 to 5, further comprising a gas-forming agent.

7. The composition according to one of claims 1 to 6, wherein said microscaffold(s) is/are between 500 µm and 1 mm in size and optionally is/are in the form of hollow beads.

8. The composition according to one of claims 1 to 7, wherein said microscaffold(s) has/have pore channel diameters of about 30 to 500 µm, preferably of about 100 to 200 µm.

9. The composition according to one of claims 1 to 8, wherein said microscaffold is coated with a biocompatible nanocrystalline calcium phosphate.

10. A composition according to one of claims 1 to 9, wherein said nanocrystalline calcium phosphate is selected from the group consisting of monocalciumphosphate anhydrite, calciumhydrogenphosphate dihydrate, dicalciumphosphate dihydrate, brushite, calciumhydrogenphosphate anhydrite, hydroxyapatite, α-tricalcium phosphate, ß-tricalcium phosphate, fluorapatite, and a combination thereof.

11. The composition according to one of claims 1 to 10, wherein said microscaffold is a calcium phosphate filled microscaffold.

12. A kit for repairing, regenerating and/or augmenting bone tissue comprising:
(a) the bioresorbable calcium phosphate material of claim 1 in one container;
(b) the biologically active agent of claim 1 in the same or a separate container; and
(c) in a further container, instructions of how to use (a) and (b).

13. A method for preparing a porous, bioresorbable, GDF releasing calcium phosphate cement according to claim 1 for use in repair, regeneration and/or augmenting of bone tissue comprising:
- providing a combination of (1) a bioresorbable calcium phosphate material which comprises at least two calcium phosphate species that, after mixing and hardening, result in a GDF-releasing calcium phosphate cement with different resorption rate(s), and (2) recombinant GDF-5 and/or GDF-3 as a biologically active agent, and (3) a bioerodible polymeric structure wherein said polymeric structure is/are microscaffold(s) having a porous structure, and (4) an aqueous liquid, preferably wherein said combination comprises a composition with the features of claim 3; and
- mixing said combination so that pores are formed upon hardening to produce a porous, bioresorbable, GDF releasing calcium phosphate cement.

14. The method according to claim 13, wherein said calcium phosphate material and the bioerodible polymeric microscaffolds and optionally gas forming agent(s) are provided in amounts that, after mixing and injecting, result in a GDF-releasing calcium phosphate cement with resorption rate(s) and GDF-releasing rates that are specifically adjusted to the age, the metabolic conditions and the kind of disease of a selected individual patient.

15. Use of a composition according to one of claims 1 to 12, for the manufacture of a medicament for use in repair, regeneration and/or augmenting of bone tissue, preferably wherein the medicament is suitable for the treatment of osteoporosis and other diseases associated with bone degeneration and/or bone fractures.

## Patentansprüche

1. Zusammensetzung zum Reparieren, Regenerieren und/oder Vermehren von Knochengewebe, wobei die Zusammensetzung rekombinante Wachstums- und Differenzierungsfaktor(en) freisetzt und ein bioresorbierbares Calciumphosphat-Material und rekombinantes GDF-5 und/oder GDF-3 als biologisch aktives Agens umfasst, **dadurch gekennzeichnet, dass**:
i) die Zusammensetzung zudem eine wässrige Flüssigkeit umfasst und injizierbar ist, so dass sie nach der Injektion zu gehärtetem Calciumphosphat (-Zement) umgebaut wird,
ii) das bioresorbierbare Calciumphosphat-Material wenigstens zwei Calciumphosphat-Arten umfasst, die verschiedene Resorptionsraten aufweisen und beim Aushärten einen Zement bilden, der Poren aufweist und Phasen umfasst, die mit verschiedenen Raten resorbiert werden,
iii) die Zusammensetzung zudem eine bioerodierbare polymere Struktur umfasst, wobei die polymere Struktur ein oder mehrere Mikrogerüst(e) mit einer porösen Struktur ist/sind.

2. Zusammensetzung nach Anspruch 1, wobei das Calciumphosphat-Material ausgewählt ist aus der Gruppe aus Calciumphosphaten (Calciumphosphat-Arten) bestehend aus Monocalciumphosphatanhydrit, Calciumhydrogenphosphatdihydrat, Dicalciumphosphatdihydrat, Brushit, Calciumhydrogenphosphatanhydrit, Hydroxyapatit, α-Tricalciumphosphat, β-Tricalciumphosphat, Fluorapatit und einer Kombination davon.

3. Zusammensetzung nach Anspruch 1 oder 2, wobei
- ein erster Anteil (I) ein Calciumphosphat enthält, das eine Löslichkeit aufweist, die als -log(Kₛ) bei 37 °C von zwischen 1 und 10 gegeben ist, und/oder
- ein zweiter Anteil (II) ein Calciumphosphat enthält, das eine Löslichkeit aufweist, die als -log(Kₛ) bei 37 °C von zwischen 10 und 50 gegeben ist, und/oder
- ein dritter Anteil (III) ein Calciumphosphat enthält, das eine Löslichkeit aufweist, die als -log(Kₛ) bei 37 °C von mehr als 50 gegeben ist, und wobei
- entweder (a) der Anteil (I) 40-100%, vorzugsweise 50-75%, mehr bevorzugt 50-60% beträgt, und der Anteil (II) 30-100%, vorzugsweise 40-60%, mehr bevorzugt 40-50% beträgt, und der Anteil (III) 0% bis 1% beträgt, wobei die resultierende Zusammensetzung zum Reparieren, Regenerieren und/oder Vermehren von Knochengewebe von Patienten mit einem Alter von 30 Jahren oder jünger geeignet ist;
- oder (b) der Anteil (I) 40-99%, vorzugsweise 50-75%, mehr bevorzugt 50-60% beträgt, und der Anteil (II) 30-99%, vorzugsweise 40-60%, mehr bevorzugt 40-50% beträgt und der Anteil (III) 1 bis 15% beträgt, wobei die resultierende Zusammensetzung zum Reparieren, Regenerieren und/oder Vermehren von Knochengewebe von Patienten mit einem Alter über 30 Jahren geeignet ist;
- oder (c) der Anteil (I) 40-84%, vorzugsweise 50-75%, mehr bevorzugt 50-60% beträgt, und der Anteil (II) 30-84%, vorzugsweise 40-60%, mehr bevorzugt 40-50% beträgt, und der Anteil (III) 16-100%, vorzugsweise 40-100% beträgt, und wobei die resultierende Zusammensetzung zum Reparieren, Regenerieren und/oder Vermehren von Knochengewebe von Patienten mit osteoporotischen Zuständen geeignet ist.

4. Zusammensetzung nach einem der Ansprüche 1 bis 3, wobei nach dem Härten eine erste Phase mit einer ersten Rate resorbiert wird und wahlweise Poren mit einem durchschnittlichen Durchmesser von weniger als einem µm aufweist, wobei das Calciumphosphat-Material vorzugsweise nach dem Härten untereinander verbundene Poren aufweist, und eine zweite Phase mit einer zweiten Rate resorbiert wird, welche die erste Rate überschreitet.

5. Zusammensetzung nach Anspruch 4, wobei die Poren Anteile der zweiten Phase umfassen und die erste Phase wahlweise im wesentlichen aus Partikeln besteht.

6. Zusammensetzung nach einem der Ansprüche 1 bis 5, die zudem ein gasbildendes Agens umfasst.

7. Zusammensetzung nach einem der Ansprüche 1 bis 6, wobei das/die Mikrogerüst(e) zwischen 500 µm und 1 mm groß ist/sind und wahlweise die Form von Hohlkugeln hat/haben.

8. Zusammensetzung nach einem der Ansprüche 1 bis 7, wobei das/die Mikrogerüst(e) Porenkanaldurchmesser von etwa 30 bis 500 µm, vorzugsweise von etwa 100 bis 200 µm hat/haben.

9. Zusammensetzung nach einem der Ansprüche 1 bis 8, wobei das Mikrogerüst mit einem biokompatiblen nanokristallinen Calciumphosphat beschichtet ist.

10. Zusammensetzung nach einem der Ansprüche 1 bis 9, wobei das nanokristalline Calciumphosphat ausgewählt ist aus der Gruppe bestehend aus Monocalciumphosphatanhydrit, Calciumhydrogenphosphatdihydrat, Dicalciumphosphatdihydrat, Brushit, Calciumhydrogenphosphatanhydrit, Hydroxyapatit, α-Tricalciumphosphat, β-Tricalciumphosphat, Fluorapatit und einer Kombination davon.

11. Zusammensetzung nach einem der Ansprüche 1 bis 10, wobei das Mikrogerüst ein mit Calciumphosphat gefülltes Mikrogerüst ist.

12. Kit zum Reparieren, Regenerieren und/oder Vermehren von Knochengewebe, umfassend:
(a) das bioresorbierbare Calciumphosphat-Material von Anspruch 1 in einem Behälter;
(b) das biologisch aktive Agens von Anspruch 1 in demselben oder einem separaten Behälter; und
(c) in einem weiteren Behälter Instruktionen, wie (a) und (b) anzuwenden sind.

13. Verfahren zum Herstellen eines porösen, bioresorbierbaren, GDF freisetzenden Calciumphosphat-Zements nach Anspruch 1 zur Verwendung beim Reparieren, Regenerieren und/oder Vermehren von Knochengewebe, umfassend:
- Bereitstellung einer Kombination aus (1) einem bioresorbierbaren Calciumphosphat-Material, das wenigstens zwei Calciumphosphat-Arten umfasst, die nach dem Mischen und Härten einen GDF-freisetzenden Calciumphosphat-Zement mit verschiedenen Resorptionsrate(n) ergeben, und (2) rekombinantes GDF-5 und/oder GDF-3 als ein biologisch aktives Agens, und (3) eine bioerodierbare polymere Struktur, wobei die polymere Struktur (ein) Mikrogerüst(e) mit poröser Struktur ist/sind, und (4) eine wässrige Flüssigkeit, wobei die Kombination vorzugsweise eine Zusammensetzung mit den Merkmalen von Anspruch 3 umfasst; und
- Mischen der Kombination, so dass beim Härten Poren geformt werden, um einen porösen, bioresorbierbaren, GDF-freisetzenden Calciumphosphat-Zement zu ergeben.

14. Verfahren nach Anspruch 13, wobei Calciumphosphat-Material und die bioerodierbaren polymeren Mikrogerüste und optional gasbildende(s) Agens in solchen Mengen bereitgestellt werden, dass sich nach dem Mischen und Injizieren ein GDF-freisetzender Calziumphosphat-Zement mit (einer) Resorptionsrate(n) und GDF-Freisetzraten ergibt, die spezifisch auf das Alter, die Stoffwechselbedingungen und die Art der Erkrankung eines ausgewählten individuellen Patienten abgestimmt sind.

15. Verwendung einer Zusammensetzung nach einem der Ansprüche 1 bis 12 zum Herstellen eines Medikaments für die Verwendung beim Reparieren, Regenerieren und/oder Vermehren von Knochengewebe, wobei das Medikament vorzugsweise zum Behandeln von Osteoporose oder anderer Erkrankungen, die mit Knochendegeneration und/oder Knochenbrüchen verbunden sind, geeignet ist.

## Revendications

1. Composition pour réparer, régénérer et/ou augmenter du tissu osseux, laquelle composition délivre un/des facteur(s) de croissance et de différenciation recombinant(s) et comprend un matériau biorésorbable de phosphate de calcium et du GDF-5 et/ou GDF-3 recombinant en tant qu'agent biologiquement actif, **caractérisée en ce que**
(i) la composition comprend en outre un liquide aqueux et peut être injectée de sorte qu'après injection, la composition est convertie en un phosphate de calcium durci (ciment),
(ii) le matériau biorésorbable de phosphate de calcium comprend au moins deux sortes de phosphate de calcium ayant des vitesses de résorption différentes et formant lors du durcissement un ciment de phosphate de calcium qui contient des pores et qui comprend des phases qui sont résorbées à des vitesses différentes,
(iii) la composition comprend en outre une structure polymérique bioérodable, ladite structure polymérique étant une/des micromatrice(s) ayant une structure poreuse.

2. Composition selon la revendication 1, dans laquelle le matériau de phosphate de calcium est choisi dans le groupe des phosphates de calcium (sortes de phosphates de calcium) composé d'anhydrite de phosphate monocalcique, d'hydrogénophosphate de calcium dihydraté, de phosphate dicalcique dihydraté, de brushite, d'anhydrite d'hydrogénophosphate de calcium, d'hydroxyapatite, de phosphate α-tricalcique, de phosphate β-tricalcique, de fluorapatite, et d'une combinaison de ceux-ci.

3. Composition selon la revendication 1 ou 2, dans laquelle
- une première portion (I) contient un phosphate de calcium ayant une solubilité exprimée sous la forme -log(Kₛ) à 37 °C comprise entre 1 et 10, et/ou
- une seconde portion (II) contient un phosphate de calcium ayant une solubilité exprimée sous la forme -log(Kₛ) à 37 °C comprise entre 10 et 50, et/ou
- une troisième portion (III) contient un phosphate de calcium ayant une solubilité exprimée sous la forme -log(Kₛ) à 37 °C supérieure 50, et
- dans laquelle soit (a) la portion (I) vaut 40-100%, de préférence 50-75%, de façon privilégiée 50-60%, et la portion (II) vaut 30-100%, de préférence 40-60%, de façon privilégiée 40-50%, et la portion (III) vaut 0% à 1%, la composition résultante convenant pour les objectifs de réparation, régénération et/ou augmentation de tissu osseux de patients dont l'âge est inférieur ou égal à 30 ans ;
- ou (b) la portion (I) vaut 40-99%, de préférence 50-75%, de façon privilégiée 50-60%, et la portion (II) vaut 30-99%, de préférence 40-60%, de façon privilégiée 40-50%, et la portion (III) vaut 1 à 15%, la composition résultante convenant pour les objectifs de réparation, régénération et/ou augmentation de tissu osseux de patients âgés de plus de 30 ans ;
- ou (c) la portion (I) vaut 40-84%, de préférence 50-75%, de façon privilégiée 50-60%, et la portion (II) vaut 30-84%, de préférence 40-60%, de façon privilégiée 40-50%, et la portion (III) vaut 16-100%, de préférence 40-100%, la composition résultante convenant pour les objectifs de réparation, régénération et/ou augmentation de tissu osseux de patients en condition ostéoporotique.

4. Composition selon l'une des revendications 1 à 3, dans laquelle, durant le durcissement, une première phase est résorbée à une première vitesse et de façon optionnelle comprend des pores ayant un diamètre moyen inférieur à un µm, ledit matériau de phosphate de calcium comprenant de préférence, durant le durcissement, des pores interconnectés, et une seconde phase est résorbée à une seconde vitesse qui dépasse ladite première vitesse.

5. Composition selon la revendication 4, dans laquelle lesdits pores comprennent des parties de ladite seconde phase et ladite première phase de façon optionnelle se compose essentiellement de particules.

6. Composition selon l'une des revendications 1 à 5, laquelle comprend en outre un agent générateur de gaz.

7. Composition selon l'une des revendications 1 à 6, dans laquelle la/les micromatrice(s) a/ont une taille comprise entre 500 µm et 1 mm et de façon optionnelle ont la forme de perles creuses.

8. Composition selon l'une des revendications 1 à 7, dans laquelle la/les micromatrice(s) a/ont des diamètres de canaux de pores d'environ 30 à 500 µm, de préférence d'environ 100 à 200 µm.

9. Composition selon l'une des revendications 1 à 8, dans laquelle ladite micromatrice est enrobée d'un phosphate de calcium nanocristallin biocompatible.

10. Composition selon l'une des revendications 1 à 9, dans laquelle ledit phosphate de calcium nanocristallin est choisi dans le groupe composé d'anhydrite de phosphate monocalcique, d'hydrogénophosphate de calcium dihydraté, de phosphate dicalcique dihydraté, de brushite, d'anhydrite d'hydrogénophosphate de calcium, d'hydroxyapatite, de phosphate α-tricalcique, de phosphate β-tricalcique, de fluorapatite, et d'une combinaison de ceux-ci.

11. Composition selon l'une des revendications 1 à 10, dans laquelle la micromatrice est une micromatrice remplie d'un phosphate de calcium.

12. Kit pour réparer, régénérer et/ou augmenter du tissu osseux, comprenant :
(a) le matériau de phosphate de calcium biorésorbable de la revendication 1 dans un contenant ;
(b) l'agent biologiquement actif de la revendication 1 dans le même contenant ou dans un contenant séparé ; et
(c) dans un autre contenant, des instructions sur la façon d'utiliser (a) et (b).

13. Méthode pour préparer un ciment de phosphate de calcium poreux, biorésorbable et libérant du GDF selon la revendication 1 pour une utilisation dans la réparation, la régénération et/ou l'augmentation de tissu osseux, comprenant :
- la fourniture d'une combinaison (1) d'un matériau de phosphate de calcium biorésorbable qui comprend au moins deux sortes de phosphates de calcium qui, après mélange et durcissement, forment un ciment de phosphate de calcium libérant du GDF avec une/des vitesse(s) de résorption différente(s), et (2) de GDF-5 et/ou GDF-3 recombinant(s) comme agent actif biologiquement, et (3) de une structure polymérique bioérodable, laquelle structure polymérique est un/des microéchafaudage(s)/micromatrice(s) ayant une structure poreuse, et (4) d'un liquide aqueux, ladite combinaison comprenant de préférence une composition ayant les caractéristiques de la revendication 3 ; et
- le mélange de ladite combinaison de sorte à former des pores durant le durcissement pour produire un ciment de phosphate de calcium poreux, biorésorbable et libérant du GDF.

14. Méthode selon la revendication 13, dans laquelle ledit matériau de phosphate de calcium et les microéchafaudages/micromatrices polymériques bioérodables et de façon optionnelle le/les agent(s) générateur(s) de gaz sont présents en une quantité telle qu'après mélange et injection, il se forme un ciment de phosphate de calcium libérant du GDF avec une/des vitesse(s) de résorption et des vitesses de libération de GDF qui sont ajustées spécifiquement à l'âge, les conditions métaboliques et le type de pathologie du patient individuel sélectionné.

15. Utilisation d'une composition selon l'une des revendications 1 à 12 pour la fabrication d'un médicament destiné à être utilisé pour la réparation, la régénération et/ou l'augmentation de tissu osseux, le médicament étant de préférence adapté pour le traitement de l'ostéoporose et d'autres pathologies associées à la dégénérescence osseuse et/ou à des fractures osseuses.
